# EUROPEAN PATENT SPECIFICATION

(11) **EP 3 137 054 B1**
(45) Date of publication and mention of the grant of the patent: **07.07.2021**
(21) Application number: 15723611.8
(22) Date of filing: 04.05.2015
(51) Int. Cl.: A61K 9/00, A61K 31/00, A61L 31/00, A61L 27/36, A61L 27/50, A61L 27/54, A61L 31/16, A61P 1/16, A61P 7/00, A61P 7/02, A61P 9/00, A61P 9/10, A61P 11/00, A61P 13/12, A61P 17/00, A61P 17/02, A61P 37/02, A61P 43/00, A61L 29/16, A61K 31/7105, A61K 31/713

(54) **COMPOSITIONS AND METHODS FOR ANTI-LYST IMMUNOMODULATION**
ZUSAMMENSETZUNGEN UND VERFAHREN ZUR ANTI-LYST-IMMUNMODULATION
COMPOSITIONS ET PROCÉDÉS D'IMMUNOMODULATION D'ANTI-LYST

(30) Priority: 02.05.2014 US 201461987910 P
(43) Date of publication of application: 08.03.2017
(73) Proprietor: Research Institute at Nationwide Children's Hospital, Columbus, Ohio 43205 (US)
(72) Inventor: BREUER, Christopher, New Albany, OH 43054 (US); HIBINO, Narutoshi, Columbus, OH 43209 (US); GARG, Vidu, Columbus, OH 43221 (US); BEST, Cameron, Columbus, OH 43122 (US)
(74) Representative: Potter Clarkson
(86) International application number: PCT/US2015/029014
(87) International publication number: WO 2015/168674

(56) References cited:
- WO-A2-99/51741
- Narutoshi Hibino ET AL: "A critical role for macrophages in neovessel formation and the development of stenosis in tissue-engineered vascular grafts", FASEB journal : official publication of the Federation of American Societies for Experimental Biology, 1 December 2011 (2011-12-01), pages 4253-4263, XP055562941, United States DOI: 10.1096/fj.11-186585 Retrieved from the Internet: URL:file:///C:\Users\SP52467\AppData\Roami ng\Mozilla\Firefox\Profiles\SP52467\CiteNP LTemp\CiteNPLWebPage.pdf [retrieved on 2019-02-28]

### SEQUENCE LISTING

<110> Nationwide Children's Hospital
   Breuer, Christopher
   Hibino, Narutoshi
   Garg, Vidu
   Best, Cameron
<120> Compositions and Methods for Anti-Lyst Immunomodulation
<130> NWCH 100 PCT
<150> US 61/987,910
   <151> 2014-05-02
<160> 2
<170> PatentIn version 3.5
<210> 1
   <211> 13503
   <212> DNA
   <213> Homo sapiens
<400> 1
   gtgcgcggcg gcggcggcag cggcgtcggc tcggggttct ccgggagagg gggagtgcgc      60
   ggcggccgca gctgccacaa accaggtgaa gctttgttct aagaatattt gtttcatcta     120
   gtttatgagt ccaaatgata tagactgtaa atgtcacagc agtggtgaaa gactgctcgg     180
   tcatgagcac cgacagtaac tcactggcac gtgaatttct gaccgatgtc aaccggcttt     240
   gcaatgcagt ggtccagagg gtggaggcca gggaggaaga agaggaggag acgcacatgg     300
   caacccttgg acagtacctt gtccatggtc gaggatttct attacttacc aagctaaatt     360
   ctataattga tcaggcattg acatgtagag aagaactcct gactcttctt ctgtctctcc     420
   ttccactggt atggaagata cctgtccaag aagaaaaggc aacagatttt aacctaccgc     480
   tctcagcaga tataatcctg accaaagaaa agaactcaag ttcacaaaga tccactcagg     540
   aaaaattaca tttagaagga agtgccctgt ctagtcaggt ttctgcaaaa gtaaatgttt     600
   ttcgaaaaag cagacgacag cgtaaaatta cccatcgcta ttctgtaaga gatgcaagaa     660
   agacacagct ctccacctca gattcagaag ccaattcaga tgaaaaaggc atagcaatga     720
   ataagcatag aaggccccat ctgctgcatc attttttaac atcgtttcct aaacaagacc     780
   accccaaagc taaacttgac cgcttagcaa ccaaagaaca gactcctcca gatgctatgg     840
   ctttggaaaa ttccagagag attattccaa gacaggggtc aaacactgac attttaagtg     900
   agccagctgc cttgtctgtt atcagtaaca tgaacaattc tccatttgac ttatgtcatg     960
   ttttgttatc tttattagaa aaagtttgta agtttgacgt taccttgaat cataattctc    1020
   ctttagcagc cagtgtagtg cccacactaa ctgaattcct agcaggcttt ggggactgct    1080
   gcagtctgag cgacaacttg gagagtcgag tagtttctgc aggttggacc gaagaaccgg    1140
   tggctttgat tcaaaggatg ctctttcgaa cagtgttgca tcttctgtca gtagatgtta 1200
   gtactgcaga gatgatgcca gaaaatctta ggaaaaattt aactgaattg cttagagcag 1260
   ctttaaaaat tagaatatgc ctagaaaagc agcctgaccc ttttgcacca agacaaaaga 1320
   aaacactgca ggaggttcag gaagattttg tgttttcaaa gtatcgtcat agagcccttc 1380
   ttttacctga gcttttggaa ggagttcttc agattctgat ctgttgtctt caaagtgcag 1440
   cttcaaatcc cttctacttc agtcaagcca tggatttggt tcaagaattc attcagcatc 1500
   atggatttaa tttatttgaa acagcagttc ttcaaatgga atggctggtt ttaagagatg 1560
   gagttcctcc cgaggcctca gagcatttga aagccctaat aaatagtgtg atgaaaataa 1620
   tgagcactgt caaaaaagtg aaatcagagc aacttcatca ttcgatgtgt acaagaaaaa 1680
   ggcacagacg atgtgaatat tctcatttta tgcatcatca ccgagatctc tcaggtcttc 1740
   tggtttcggc ttttaaaaac caggtttcca aaaacccatt tgaagagact gcagatggag 1800
   atgtttatta tcctgagcgg tgctgttgca ttgcagtgtg tgcccatcag tgcttgcgct 1860
   tactacagca ggcttccttg agcagcactt gtgtccagat cctatcgggt gttcataaca 1920
   ttggaatatg ctgttgtatg gatcccaaat ctgtaatcat tcctttgctc catgctttta 1980
   aattgccagc actgaaaaat tttcagcagc atatattgaa tatccttaac aaacttattt 2040
   tggatcagtt aggaggagca gagatatcac caaaaattaa aaaagcagct tgtaatattt 2100
   gtactgttga ctctgaccaa ctagcccaat tagaagagac actgcaggga aacttatgtg 2160
   atgctgaact ctcctcaagt ttatccagtc cttcttacag atttcaaggg atcctgccca 2220
   gcagtggatc tgaagatttg ttgtggaaat gggatgcttt aaaggcttat cagaactttg 2280
   tttttgaaga agacagatta catagtatac agattgcaaa tcacatttgc aatttaatcc 2340
   agaaaggcaa tatagttgtt cagtggaaat tatataatta catatttaat cctgtgctcc 2400
   aaagaggagt tgaattagca catcattgtc aacacctaag cgttacttca gctcaaagtc 2460
   atgtatgtag ccatcataac cagtgcttgc ctcaggacgt gcttcagatt tatgtaaaaa 2520
   ctctgcctat cctgcttaaa tccagggtaa taagagattt gtttttgagt tgtaatggag 2580
   taagtcaaat aatcgaatta aattgcttaa atggtattcg aagtcattct ctaaaagcat 2640
   ttgaaactct gataatcagc ctaggggagc aacagaaaga tgcctcagtt ccagatattg 2700
   atgggataga cattgaacag aaggagttgt cctctgtaca tgtgggtact tcttttcatc 2760
   atcagcaagc ttattcagat tctcctcaga gtctcagcaa attttatgct ggcctcaaag 2820
   aagcttatcc aaagagacgg aagactgtta accaagatgt tcatatcaac acaataaacc 2880
   tattcctctg tgtggctttt ttatgcgtaa gtaaagaagc agagtctgac agggagtcgg 2940
   ccaatgactc agaagatact tctggctatg acagcacagc cagcgagcct ttaagtcata 3000
   tgctgccatg tatatctctc gagagccttg tcttgccttc tcctgaacat atgcaccaag 3060
   cagcagacat ttggtctatg tgtcgttgga tctacatgtt gagttcagtg ttccagaaac 3120
   agttttatag gcttggtggt ttccgagtat gccataagtt aatatttatg ataatacaga 3180
   aactgttcag aagtcacaaa gaggagcaag gaaaaaagga gggagataca agtgtaaatg 3240
   aaaaccagga tttaaacaga atttctcaac ctaagagaac tatgaaggaa gatttattat 3300
   ctttggctat aaaaagtgac cccataccat cagaactagg tagtctaaaa aagagtgctg 3360
   acagtttagg taaattagag ttacagcata tttcttccat aaatgtggaa gaagtttcag 3420
   ctactgaagc cgctcccgag gaagcaaagc tatttacaag tcaagaaagt gagacctcac 3480
   ttcaaagtat acgacttttg gaagcccttc tggccatttg tcttcatggt gccagaacta 3540
   gtcaacagaa gatggaattg gagttaccta atcagaactt gtctgtggaa agtatattat 3600
   ttgaaatgag ggaccatctt tcccagtcaa aggtgattga aacacaacta gcaaagcctt 3660
   tatttgatgc cctgcttcga gttgccctcg ggaattattc agcagatttt gaacataatg 3720
   atgctatgac tgagaagagt catcaatctg cagaagaatt gtcatcccag cctggtgatt 3780
   tttcagaaga agctgaggat tctcagtgtt gtagttttaa acttttagtt gaagaagaag 3840
   gttacgaagc agatagtgaa agcaatcctg aagatggcga aacccaggat gatggggtag 3900
   acttaaagtc tgaaacagaa ggtttcagtg catcaagcag tccaaatgac ttactcgaaa 3960
   acctcactca aggggaaata atttatcctg agatttgtat gctggaatta aatttgcttt 4020
   ctgctagtaa agccaaactt gatgtgcttg cccatgtatt tgagagtttt ttgaaaatta 4080
   ttaggcagaa agaaaagaat gtttttctgc tcatgcaaca gggaactgtg aaaaatcttt 4140
   taggagggtt cttgagtatt ttaacacagg atgattctga ttttcaagca tgccagagag 4200
   tattggtgga tcttttggta tctttgatga gttcaagaac atgttcagaa gagctaaccc 4260
   ttcttttgag aatatttctg gagaaatctc cttgtacaaa aattcttctt ctgggtattc 4320
   tgaaaattat tgaaagtgat actactatga gcccttcaca gtatctaacc ttccctttac 4380
   tgcacgctcc aaatttaagc aacggtgttt catcacaaaa gtatcctggg attttaaaca 4440
   gtaaggccat gggtttattg agaagagcac gagtttcacg gagcaagaaa gaggctgata 4500
   gagagagttt tccccatcgg ctgctttcat cttggcacat agccccagtc cacctgccgt 4560
   tgctggggca aaactgctgg ccacacctat cagaaggttt cagtgtttcc ctgtggttta 4620
   atgtggagtg tatccatgaa gctgagagta ctacagaaaa aggaaagaag ataaagaaaa 4680
   gaaacaaatc attaatttta ccagatagca gttttgatgg tacagagagc gacagaccag 4740
   aaggtgcaga gtacataaat cctggtgaaa gactcataga agaaggatgt attcatataa 4800
   tttcactggg atccaaagcg ttgatgatcc aagtgtgggc tgatccccac aatgccactc 4860
   ttatctttcg tgtgtgcatg gattcaaatg atgacatgaa agctgtttta ctagcacagg 4920
   ttgaatcaca ggagaatatt ttcctcccaa gcaaatggca acatttagta ctcacctact 4980
   tacagcagcc ccaagggaaa aggaggattc atgggaaaat ctccatatgg gtctctggac 5040
   agaggaagcc tgatgttact ttggatttta tgcttccaag aaaaacaagt ttgtcatctg 5100
   atagcaataa aacattttgc atgattggcc attgtttatc atcccaagaa gagtttttgc 5160
   agttggctgg aaaatgggac ctgggaaatt tgcttctctt caacggagct aaggttggtt 5220
   cacaagaggc cttttatctg tatgcttgtg gacccaacca tacatctgta atgccatgta 5280
   agtatggcaa gccagtcaat gactactcca aatatattaa taaagaaatt ttgcgatgtg 5340
   aacaaatcag agaacttttt atgaccaaga aagatgtgga tattggtctc ttaattgaaa 5400
   gtctttcagt tgtttataca acttactgtc ctgctcagta taccatctat gaaccagtga 5460
   ttagacttaa aggtcaaatg aaaacccaac tctctcaaag acccttcagc tcaaaagaag 5520
   ttcagagcat cttattagaa cctcatcatc taaagaatct ccaacctact gaatataaaa 5580
   ctattcaagg cattctgcac gaaattggtg gaactggcat atttgttttt ctctttgcca 5640
   gggttgttga actcagtagc tgtgaagaaa ctcaagcatt agcactgcga gttatactct 5700
   cattaattaa atacaaccaa caaagagtac atgaattaga aaattgtaat ggactttcta 5760
   tgattcatca ggtgttgatc aaacaaaaat gcattgttgg gttttacatt ttgaagaccc 5820
   ttcttgaagg atgctgtggt gaagatatta tttatatgaa tgagaatgga gagtttaagt 5880
   tggatgtaga ctctaatgct ataatccaag atgttaagct gttagaggaa ctattgcttg 5940
   actggaagat atggagtaaa gcagagcaag gtgtttggga aactttgcta gcagctctag 6000
   aagtcctcat cagagcagat caccaccagc agatgtttaa tattaagcag ttattgaaag 6060
   ctcaagtggt tcatcacttt ctactgactt gtcaggtttt gcaggaatac aaagaggggc 6120
   aactcacacc catgccccga gaggtttgta gatcatttgt gaaaattata gcagaagtcc 6180
   ttggatctcc tccagatttg gaattattga caattatctt caatttcctt ttagcagttc 6240
   accctcctac taatacttac gtttgtcaca atcccacgaa cttctacttt tctttgcaca 6300
   tagatggcaa gatctttcag gagaaagtgc ggtcaatcat gtacctgagg cattccagca 6360
   gtggaggaag gtcccttatg agccctggat ttatggtaat aagcccatct ggttttactg 6420
   cttcaccata tgaaggagag aattcctcta atattattcc acaacagatg gccgcccata 6480
   tgctgcgttc tagaagccta ccagcattcc ctacttcttc actactaacg caatcacaaa 6540
   aactgactgg aagtttgggt tgtagtatcg acaggttaca aaatattgca gatacttatg 6600
   ttgccaccca atcaaagaaa caaaattctt tggggagttc cgacacactg aaaaaaggca 6660
   aagaggacgc attcatcagt agctgtgagt ctgcaaaaac tgtttgtgaa atggaagctg 6720
   tcctctcagc ccaggtctct gtcagtgatg tcccaaaggg agtgctggga tttccagtgg 6780
   tcaaagcaga tcataaacag ttgggagcag aacccaggtc agaagatgac agtcctgggg 6840
   atgagtcctg cccacgccga cctgattacc taaagggatt ggcctccttc cagcgaagcc 6900
   acagcactat tgcaagcctt gggctagctt ttccttcaca gaacggatct gcagctgttg 6960
   gccgttggcc aagtcttgtt gatagaaaca ctgatgattg ggaaaacttt gcctattctc 7020
   ttggttatga gccaaattac aaccgaactg caagtgctca cagtgtaact gaagactgtt 7080
   tggtacctat atgctgtgga ttatatgaac tcctaagtgg ggttcttctt atcctgcctg 7140
   atgttttgct tgaagatgtg atggacaagc ttattcaagc agatacactt ttggtcctcg 7200
   ttaaccaccc atcaccagct atacaacaag gtgttattaa actattagat gcatattttg 7260
   ctagagcatc taaggaacaa aaagataaat ttctgaagaa tcgtggattt tccttgctag 7320
   ccaaccagtt gtatcttcat cgaggaactc aagaattgtt agaatgcttc atcgaaatgt 7380
   tctttggtcg acatattggc cttgatgaag aatttgatct ggaagatgtg agaaacatgg 7440
   gattgtttca gaagtggtct gtcattccta ttctgggact aatagagacc tctctatatg 7500
   acaacatact cttgcataat gctcttttac ttcttctcca aattttaaat tcttgttcta 7560
   aggtagcaga tatgttgctg gataatggtc tactctatgt gttatgtaat acagtagcag 7620
   ccctgaatgg attagaaaag aacattccca tgagtgaata taaattgctt gcttgtgata 7680
   tacagcaact tttcatagca gttacaattc atgcttgcag ttcctcaggc tcacaatatt 7740
   ttagggttat tgaagacctt attgtaatgc ttggatatct tcaaaatagc aaaaacaaga 7800
   ggacacaaaa tatggctgtt gcactacagc ttagagttct ccaggctgct atggaattta 7860
   taaggaccac cgcaaatcat gactctgaaa acctcacaga ttcactccag tcaccttctg 7920
   ctccccatca tgcagtagtt caaaagcgga aaagcattgc tggtcctcga aaatttcccc 7980
   ttgctcaaac tgaatcgctt ctgatgaaaa tgcgttcagt ggcaaatgat gagcttcatg 8040
   tgatgatgca acggagaatg agccaagaga accctagcca agcaactgaa acggaacttg 8100
   cgcagagact acagaggctc actgttttag cagtcaacag gattatttat caagaattta 8160
   attcagacat tattgacatt ttgagaactc cagaaaatgt aactcaaagc aagacctcag 8220
   ttttccagac cgaaatttct gaggaaaata ttcatcatga acagtcttct gttttcaatc 8280
   catttcagaa agaaattttt acatatctgg tagaaggatt caaagtatct attggttcaa 8340
   gtaaagccag tggttccaag cagcaatgga ctaaaattct gtggtcttgt aaggagacct 8400
   tccgaatgca gcttgggaga ctactagtgc atattttgtc gccagcccac gctgcacaag 8460
   agagaaagca aatttttgaa atagttcatg aaccaaatca tcaggaaata ctacgagact 8520
   gtctcagccc atccctacaa catggagcca agttagtttt gtatttgtca gagttgatac 8580
   ataatcacca aggtgaattg actgaagaag agctaggcac agcagaactg cttatgaatg 8640
   ctttgaagtt atgtggtcac aagtgcatcc ctcccagtgc atcaacaaaa gcagacctta 8700
   ttaaaatgat caaagaggaa caaaagaaat atgaaactga agaaggagtg aataaagctg 8760
   cttggcagaa aacagttaac aataatcaac aaagtctctt tcagcgtctg gattcaaaat 8820
   caaaggatat atctaaaata gctgcagata tcacccaggc agtgtctctc tcccaaggaa 8880
   atgagagaaa aaaggtgatc cagcatatta gaggaatgta taaagtagat ttgagtgcca 8940
   gcagacattg gcaggaactt attcagcagc tgacacatga tagagcagta tggtatgacc 9000
   ccatctacta tccaacctca tggcagttgg atccaacaga agggccaaat cgagagagga 9060
   gacgtttaca gagatgttat ttaactattc caaataagta tctccttagg gatagacaga 9120
   aatcagaaga tgttgtcaaa ccaccactct cttacctgtt tgaagacaaa actcattctt 9180
   ctttctcttc tactgtcaaa gacaaagctg caagtgaatc tataagagtg aatcgaagat 9240
   gcatcagtgt tgcaccatct agagagacag ctggtgaatt gttactaggt aaatgtggaa 9300
   tgtattttgt ggaagataat gcttctgata cagttgaaag ttcgagcctt cagggagagt 9360
   tggaaccagc atcattttcc tggacatatg aagaaattaa agaagttcac aagcgttggt 9420
   ggcaattgag agataatgct gtagaaatct ttctaacaaa tggcagaaca ctcctgttgg 9480
   catttgataa caccaaggtt cgtgatgatg tataccacaa tatactcaca aataacctcc 9540
   ctaatcttct ggaatatggt aacatcaccg ctctgacaaa tttatggtat actgggcaaa 9600
   ttactaattt tgaatatttg actcacttaa acaaacatgc tggccgatcc ttcaatgatc 9660
   tcatgcagta tcctgtgttc ccatttatac ttgctgacta cgttagtgag acacttgacc 9720
   tcaatgatct gttgatatac agaaatctct ctaaacctat agctgttcag tataaagaaa 9780
   aagaagatcg ttatgtggac acatacaagt acttggagga agagtaccgc aaaggagcca 9840
   gagaagatga ccccatgcct cccgtgcagc cctatcacta tggctcccac tattccaata 9900
   gcggcactgt gcttcacttc ctggtcagga tgcctccttt cactaaaatg tttttagcct 9960
   atcaagatca aagttttgac attccagaca gaacttttca ttctacaaat acaacttggc 10020
   gactctcatc ttttgaatct atgactgatg tgaaagaact tatcccagag tttttctatc 10080
   ttccagagtt cctagttaac cgtgaaggtt ttgattttgg tgtgcgtcag aatggtgaac 10140
   gggttaatca cgtcaacctt ccccctgggg cgcgtaatga tcctcgtctt tttatcctca 10200
   tccatcggca ggctctagag tctgactacg tgtcgcagaa catctgtcag tggattgact 10260
   tggtgtttgg gtataagcaa aaggggaagg cttctgttca agcgatcaat gtttttcatc 10320
   ctgctacata ttttggaatg gatgtctctg cagttgaaga tccagttcag agacgagcgc 10380
   tagaaaccat gataaaaacc tacgggcaga ctccccgtca gctgttccac atggcccatg 10440
   tgagcagacc tggagccaag ctcaatattg aaggagagct tccagctgct gtggggttgc 10500
   tagtgcagtt tgctttcagg gagacccgag aacaggtcaa agaaatcacc tatccgagtc 10560
   ctttgtcatg gataaaaggc ttgaaatggg gggaatacgt gggttccccc agtgctccag 10620
   tacctgtggt ctgcttcagc cagccccacg gagaaagatt tggctctctc caggctctgc 10680
   ccaccagagc aatctgtggt ttgtcacgga atttctgtct tctgatgaca tatagcaagg 10740
   aacaaggtgt gagaagcatg aacagtacgg acattcagtg gtcagccatc ctgagctggg 10800
   gatatgctga taatatttta aggttgaaga gtaaacaaag tgagcctcca gtaaacttta 10860
   ttcaaagttc acaacagtac caggtgacta gttgtgcttg ggtgcctgac agttgccagc 10920
   tgtttactgg aagcaaatgc ggtgtcatca cagcctacac aaacagattt acaagcagca 10980
   cgccatcaga aatagaaatg gagactcaaa tacatctcta tggtcacaca gaagagataa 11040
   ccagcttatt tgtttgcaaa ccatacagta tactgataag tgtgagcaga gacggaacct 11100
   gcatcatatg ggatttaaac aggttatgct atgtacaaag tctggcggga cacaaaagcc 11160
   ctgtcacagc tgtctctgcc agtgaaacct caggtgatat tgctactgtg tgtgattcag 11220
   ctggcggagg cagtgacctc agactctgga cggtgaacgg ggatctcgtt ggacatgtcc 11280
   actgcaggga gatcatctgt tccgtggctt tctccaacca gcctgaggga gtatctatca 11340
   atgtaatcgc tgggggatta gaaaatggaa ttgtaaggtt atggagcaca tgggacttaa 11400
   agcctgtgag agaaattaca tttcccaaat caaataagcc catcatcagc cttacatttt 11460
   cttgtgatgg ccaccatttg tacacagcaa acagtgatgg gaccgtgatt gcctggtgtc 11520
   ggaaggacca gcagcgcttg aaacagccaa tgttctattc cttccttagc agctatgcag 11580
   ccgggtgaat gcgaatgaac ttcacgttct ccaaagcact ttaactccaa actagatttg 11640
   ttgacttcac cagttttagg aggttgaacc taaagaaatg gatgactgga caaaccatcc 11700
   aaataatgat aaagtctatt catctgcaca aaattctgaa gagtcacatg atcctaagag 11760
   gaaagttctg ttctatttta gtgataatct ggaagattgt gtcaatatgc actagccaac 11820
   aagttttaag cctcgcatgg tacattaaaa tgatattctt aaaatttttt cccaccaagg 11880
   tattccaaag aaaatattaa ggtctcccct ttttctatga ttccaaaagg accagtagaa 11940
   tttaaattgg ttggttgatt gtttatataa aacacactaa aattatattt taaaagttta 12000
   ctgccatgaa atactcctcc caccacacac acatgctcca aaagaggaaa gaaaaaaaga 12060
   taatttttag gacttgataa ttgctttctt tgagaagcaa attattcagt aggtgcctct 12120
   gtaccaaata ttttatggaa tatctaaata ctaaaataaa ctatgaatga atctcaaaat 12180
   taggcagttt ttgccagttg ctttcttagc tcaaaggaga accagaattt ttttgacagc 12240
   cacaaacaag aatacaggta tcttggattt cagacacatt ctgtttcttc ataaaaattt 12300
   tacttaaaat ctgtaacgct agatattgac tatccttagt tgagtcactg aggtttaaac 12360
   acaatggtaa gtcttaaagt ctgctattta cagagcattg aatctgtacc aatttgcaat 12420
   agaaagcctt cagtatgcaa gaagtttgca tgggtattaa gaacacagcc taaataaggc 12480
   atttgatcta atctgcagga agaattttct tccccaaaac agaattataa aagcttactt 12540
   taaacaggag gcagaataat tcttttagga aaccatttca ttctgtttct actaacctat 12600
   accatctgag aattctagga agaataataa aatctcgtgt attccacagc aaacttacat 12660
   accataaaga cagaattcct aaacatcttg gagccgtctg tctctcccat atgatggctg 12720
   tctgtatatt tttacttggg gtgctgcttt attggctttg aaaacactgt cagataagct 12780
   cagtaatatg ttaccatggg ataaaaatat gtatccctgc ctaagaataa cttgtgcatt 12840
   tgttatggaa atttaattca tatggtgttt acagtactac ttttgtaact tccagacttt 12900
   ctaaaacatt ctgcttaaaa accatataaa atataattcc aaagtctctg ctgtcaagat 12960
   agattcgaga gaaagcacgt ggccatgtat gctttaacct taaactgcat acacatgtag 13020
   tgatacctag gctgcattta gatcaccgtg tgctcaggcc aggtgtgaat cctgaggtcc 13080
   atggaggtgc agagatgaga ttactcctat tcacgttgaa gtgatttgct ttgttaacaa 13140
   aaaattgcag ctattgtcta gctttcattt ttttactgag aactttaaat tagtccccta 13200
   ttagaatagg gttgctactc atcttttttt aaaaaccgaa tttcatcatt tatctaaaga 13260
   gaaaatatgc agaataactg gtcttgttaa gagtgcaata ttatattttt atgtaaaaat 13320
   aaaaattaat ttggggggat tatttattca gcatgaaacc taatatgtat atgtttgaaa 13380
   tacttcataa tgtgcatgtt gtagcaaaca tttctgtaaa ttatcacaag ctctgttacc 13440
   tttatataca ctgcctcttc aatttggaaa taaatttcat aaaaatagat gtaaacaaaa 13500
   aaa 13503
<210> 2
   <211> 3801
   <212> PRT
   <213> Homo sapiens
<400> 2
   Met Ser Thr Asp Ser Asn Ser Leu Ala Arg Glu Phe Leu Thr Asp Val
   1 5 10 15
   Asn Arg Leu Cys Asn Ala Val Val Gln Arg Val Glu Ala Arg Glu Glu
   20 25 30
   Glu Glu Glu Glu Thr His Met Ala Thr Leu Gly Gln Tyr Leu Val His
   35 40 45
   Gly Arg Gly Phe Leu Leu Leu Thr Lys Leu Asn Ser Ile Ile Asp Gln
   50 55 60
   Ala Leu Thr Cys Arg Glu Glu Leu Leu Thr Leu Leu Leu Ser Leu Leu
   65 70 75 80
   Pro Leu Val Trp Lys Ile Pro Val Gln Glu Glu Lys Ala Thr Asp Phe
   85 90 95
   Asn Leu Pro Leu Ser Ala Asp Ile Ile Leu Thr Lys Glu Lys Asn Ser
   100 105 110
   Ser Ser Gln Arg Ser Thr Gln Glu Lys Leu His Leu Glu Gly Ser Ala
   115 120 125
   Leu Ser Ser Gln Val Ser Ala Lys Val Asn Val Phe Arg Lys Ser Arg
   130 135 140
   Arg Gln Arg Lys Ile Thr His Arg Tyr Ser Val Arg Asp Ala Arg Lys
   145 150 155 160
   Thr Gln Leu Ser Thr Ser Asp Ser Glu Ala Asn Ser Asp Glu Lys Gly
   165 170 175
   Ile Ala Met Asn Lys His Arg Arg Pro His Leu Leu His His Phe Leu
   180 185 190
   Thr Ser Phe Pro Lys Gln Asp His Pro Lys Ala Lys Leu Asp Arg Leu
   195 200 205
   Ala Thr Lys Glu Gln Thr Pro Pro Asp Ala Met Ala Leu Glu Asn Ser
   210 215 220
   Arg Glu Ile Ile Pro Arg Gln Gly Ser Asn Thr Asp Ile Leu Ser Glu
   225 230 235 240
   Pro Ala Ala Leu Ser Val Ile Ser Asn Met Asn Asn Ser Pro Phe Asp
   245 250 255
   Leu Cys His Val Leu Leu Ser Leu Leu Glu Lys Val Cys Lys Phe Asp
   260 265 270
   Val Thr Leu Asn His Asn Ser Pro Leu Ala Ala Ser Val Val Pro Thr
   275 280 285
   Leu Thr Glu Phe Leu Ala Gly Phe Gly Asp Cys Cys Ser Leu Ser Asp
   290 295 300
   Asn Leu Glu Ser Arg Val Val Ser Ala Gly Trp Thr Glu Glu Pro Val
   305 310 315 320
   Ala Leu Ile Gln Arg Met Leu Phe Arg Thr Val Leu His Leu Leu Ser
   325 330 335
   Val Asp Val Ser Thr Ala Glu Met Met Pro Glu Asn Leu Arg Lys Asn
               340                 345                 350
   Leu Thr Glu Leu Leu Arg Ala Ala Leu Lys Ile Arg Ile Cys Leu Glu
           355                 360                 365
   Lys Gln Pro Asp Pro Phe Ala Pro Arg Gln Lys Lys Thr Leu Gln Glu
       370                 375                 380
   Val Gln Glu Asp Phe Val Phe Ser Lys Tyr Arg His Arg Ala Leu Leu
   385                 390                 395                 400
   Leu Pro Glu Leu Leu Glu Gly Val Leu Gln Ile Leu Ile Cys Cys Leu
                   405                 410                 415
   Gln Ser Ala Ala Ser Asn Pro Phe Tyr Phe Ser Gln Ala Met Asp Leu
               420                 425                 430
   Val Gln Glu Phe Ile Gln His His Gly Phe Asn Leu Phe Glu Thr Ala
           435                 440                 445
   Val Leu Gln Met Glu Trp Leu Val Leu Arg Asp Gly Val Pro Pro Glu
       450                 455                 460
   Ala Ser Glu His Leu Lys Ala Leu Ile Asn Ser Val Met Lys Ile Met
   465                 470                 475                 480
   Ser Thr Val Lys Lys Val Lys Ser Glu Gln Leu His His Ser Met Cys
                   485                 490                 495
   Thr Arg Lys Arg His Arg Arg Cys Glu Tyr Ser His Phe Met His His
               500                 505                 510
   His Arg Asp Leu Ser Gly Leu Leu Val Ser Ala Phe Lys Asn Gln Val
           515                 520                 525
   Ser Lys Asn Pro Phe Glu Glu Thr Ala Asp Gly Asp Val Tyr Tyr Pro
       530                 535                 540
   Glu Arg Cys Cys Cys Ile Ala Val Cys Ala His Gln Cys Leu Arg Leu
   545                 550                 555                 560
   Leu Gln Gln Ala Ser Leu Ser Ser Thr Cys Val Gln Ile Leu Ser Gly
                   565                 570                 575
   Val His Asn Ile Gly Ile Cys Cys Cys Met Asp Pro Lys Ser Val Ile
   580 585 590
   Ile Pro Leu Leu His Ala Phe Lys Leu Pro Ala Leu Lys Asn Phe Gln
   595 600 605
   Gln His Ile Leu Asn Ile Leu Asn Lys Leu Ile Leu Asp Gln Leu Gly
   610 615 620
   Gly Ala Glu Ile Ser Pro Lys Ile Lys Lys Ala Ala Cys Asn Ile Cys
   625 630 635 640
   Thr Val Asp Ser Asp Gln Leu Ala Gln Leu Glu Glu Thr Leu Gln Gly
   645 650 655
   Asn Leu Cys Asp Ala Glu Leu Ser Ser Ser Leu Ser Ser Pro Ser Tyr
   660 665 670
   Arg Phe Gln Gly Ile Leu Pro Ser Ser Gly Ser Glu Asp Leu Leu Trp
   675 680 685
   Lys Trp Asp Ala Leu Lys Ala Tyr Gln Asn Phe Val Phe Glu Glu Asp
   690 695 700
   Arg Leu His Ser Ile Gln Ile Ala Asn His Ile Cys Asn Leu Ile Gln
   705 710 715 720
   Lys Gly Asn Ile Val Val Gln Trp Lys Leu Tyr Asn Tyr Ile Phe Asn
   725 730 735
   Pro Val Leu Gln Arg Gly Val Glu Leu Ala His His Cys Gln His Leu
   740 745 750
   Ser Val Thr Ser Ala Gln Ser His Val Cys Ser His His Asn Gln Cys
   755 760 765
   Leu Pro Gln Asp Val Leu Gln Ile Tyr Val Lys Thr Leu Pro Ile Leu
   770 775 780
   Leu Lys Ser Arg Val Ile Arg Asp Leu Phe Leu Ser Cys Asn Gly Val
   785 790 795 800
   Ser Gln Ile Ile Glu Leu Asn Cys Leu Asn Gly Ile Arg Ser His Ser
   805 810 815
   Leu Lys Ala Phe Glu Thr Leu Ile Ile Ser Leu Gly Glu Gln Gln Lys
   820 825 830
   Asp Ala Ser Val Pro Asp Ile Asp Gly Ile Asp Ile Glu Gln Lys Glu
   835 840 845
   Leu Ser Ser Val His Val Gly Thr Ser Phe His His Gln Gln Ala Tyr
   850 855 860
   Ser Asp Ser Pro Gln Ser Leu Ser Lys Phe Tyr Ala Gly Leu Lys Glu
   865 870 875 880
   Ala Tyr Pro Lys Arg Arg Lys Thr Val Asn Gln Asp Val His Ile Asn
   885 890 895
   Thr Ile Asn Leu Phe Leu Cys Val Ala Phe Leu Cys Val Ser Lys Glu
   900 905 910
   Ala Glu Ser Asp Arg Glu Ser Ala Asn Asp Ser Glu Asp Thr Ser Gly
   915 920 925
   Tyr Asp Ser Thr Ala Ser Glu Pro Leu Ser His Met Leu Pro Cys Ile
   930 935 940
   Ser Leu Glu Ser Leu Val Leu Pro Ser Pro Glu His Met His Gln Ala
   945 950 955 960
   Ala Asp Ile Trp Ser Met Cys Arg Trp Ile Tyr Met Leu Ser Ser Val
   965 970 975
   Phe Gln Lys Gln Phe Tyr Arg Leu Gly Gly Phe Arg Val Cys His Lys
   980 985 990
   Leu Ile Phe Met Ile Ile Gln Lys Leu Phe Arg Ser His Lys Glu Glu
   995 1000 1005
   Gln Gly Lys Lys Glu Gly Asp Thr Ser Val Asn Glu Asn Gln Asp
   1010 1015 1020
   Leu Asn Arg Ile Ser Gln Pro Lys Arg Thr Met Lys Glu Asp Leu
   1025 1030 1035
   Leu Ser Leu Ala Ile Lys Ser Asp Pro Ile Pro Ser Glu Leu Gly
   1040 1045 1050
   Ser Leu Lys Lys Ser Ala Asp Ser Leu Gly Lys Leu Glu Leu Gln
   1055 1060 1065
   His Ile Ser Ser Ile Asn Val Glu Glu Val Ser Ala Thr Glu Ala
   1070 1075 1080
   Ala Pro Glu Glu Ala Lys Leu Phe Thr Ser Gln Glu Ser Glu Thr
   1085 1090 1095
   Ser Leu Gln Ser Ile Arg Leu Leu Glu Ala Leu Leu Ala Ile Cys
   1100 1105 1110
   Leu His Gly Ala Arg Thr Ser Gln Gln Lys Met Glu Leu Glu Leu
   1115 1120 1125
   Pro Asn Gln Asn Leu Ser Val Glu Ser Ile Leu Phe Glu Met Arg
   1130 1135 1140
   Asp His Leu Ser Gln Ser Lys Val Ile Glu Thr Gln Leu Ala Lys
   1145 1150 1155
   Pro Leu Phe Asp Ala Leu Leu Arg Val Ala Leu Gly Asn Tyr Ser
   1160 1165 1170
   Ala Asp Phe Glu His Asn Asp Ala Met Thr Glu Lys Ser His Gln
   1175 1180 1185
   Ser Ala Glu Glu Leu Ser Ser Gln Pro Gly Asp Phe Ser Glu Glu
   1190 1195 1200
   Ala Glu Asp Ser Gln Cys Cys Ser Phe Lys Leu Leu Val Glu Glu
   1205 1210 1215
   Glu Gly Tyr Glu Ala Asp Ser Glu Ser Asn Pro Glu Asp Gly Glu
   1220 1225 1230
   Thr Gln Asp Asp Gly Val Asp Leu Lys Ser Glu Thr Glu Gly Phe
   1235 1240 1245
   Ser Ala Ser Ser Ser Pro Asn Asp Leu Leu Glu Asn Leu Thr Gln
   1250 1255 1260
   Gly Glu Ile Ile Tyr Pro Glu Ile Cys Met Leu Glu Leu Asn Leu
   1265 1270 1275
   Leu Ser Ala Ser Lys Ala Lys Leu Asp Val Leu Ala His Val Phe
   1280 1285 1290
   Glu Ser Phe Leu Lys Ile Ile Arg Gln Lys Glu Lys Asn Val Phe
   1295 1300 1305
   Leu Leu Met Gln Gln Gly Thr Val Lys Asn Leu Leu Gly Gly Phe
   1310 1315 1320
   Leu Ser Ile Leu Thr Gln Asp Asp Ser Asp Phe Gln Ala Cys Gln
   1325 1330 1335
   Arg Val Leu Val Asp Leu Leu Val Ser Leu Met Ser Ser Arg Thr
   1340 1345 1350
   Cys Ser Glu Glu Leu Thr Leu Leu Leu Arg Ile Phe Leu Glu Lys
   1355 1360 1365
   Ser Pro Cys Thr Lys Ile Leu Leu Leu Gly Ile Leu Lys Ile Ile
   1370 1375 1380
   Glu Ser Asp Thr Thr Met Ser Pro Ser Gln Tyr Leu Thr Phe Pro
   1385 1390 1395
   Leu Leu His Ala Pro Asn Leu Ser Asn Gly Val Ser Ser Gln Lys
   1400 1405 1410
   Tyr Pro Gly Ile Leu Asn Ser Lys Ala Met Gly Leu Leu Arg Arg
   1415 1420 1425
   Ala Arg Val Ser Arg Ser Lys Lys Glu Ala Asp Arg Glu Ser Phe
   1430 1435 1440
   Pro His Arg Leu Leu Ser Ser Trp His Ile Ala Pro Val His Leu
   1445 1450 1455
   Pro Leu Leu Gly Gln Asn Cys Trp Pro His Leu Ser Glu Gly Phe
   1460 1465 1470
   Ser Val Ser Leu Trp Phe Asn Val Glu Cys Ile His Glu Ala Glu
   1475 1480 1485
   Ser Thr Thr Glu Lys Gly Lys Lys Ile Lys Lys Arg Asn Lys Ser
   1490 1495 1500
   Leu Ile Leu Pro Asp Ser Ser Phe Asp Gly Thr Glu Ser Asp Arg
   1505 1510 1515
   Pro Glu Gly Ala Glu Tyr Ile Asn Pro Gly Glu Arg Leu Ile Glu
   1520 1525 1530
   Glu Gly Cys Ile His Ile Ile Ser Leu Gly Ser Lys Ala Leu Met
   1535 1540 1545
   Ile Gln Val Trp Ala Asp Pro His Asn Ala Thr Leu Ile Phe Arg
   1550 1555 1560
   Val Cys Met Asp Ser Asn Asp Asp Met Lys Ala Val Leu Leu Ala
   1565 1570 1575
   Gln Val Glu Ser Gln Glu Asn Ile Phe Leu Pro Ser Lys Trp Gln
   1580 1585 1590
   His Leu Val Leu Thr Tyr Leu Gln Gln Pro Gln Gly Lys Arg Arg
   1595 1600 1605
   Ile His Gly Lys Ile Ser Ile Trp Val Ser Gly Gln Arg Lys Pro
   1610 1615 1620
   Asp Val Thr Leu Asp Phe Met Leu Pro Arg Lys Thr Ser Leu Ser
   1625 1630 1635
   Ser Asp Ser Asn Lys Thr Phe Cys Met Ile Gly His Cys Leu Ser
   1640 1645 1650
   Ser Gln Glu Glu Phe Leu Gln Leu Ala Gly Lys Trp Asp Leu Gly
   1655 1660 1665
   Asn Leu Leu Leu Phe Asn Gly Ala Lys Val Gly Ser Gln Glu Ala
   1670 1675 1680
   Phe Tyr Leu Tyr Ala Cys Gly Pro Asn His Thr Ser Val Met Pro
   1685 1690 1695
   Cys Lys Tyr Gly Lys Pro Val Asn Asp Tyr Ser Lys Tyr Ile Asn
   1700 1705 1710
   Lys Glu Ile Leu Arg Cys Glu Gln Ile Arg Glu Leu Phe Met Thr
   1715 1720 1725
   Lys Lys Asp Val Asp Ile Gly Leu Leu Ile Glu Ser Leu Ser Val
   1730 1735 1740
   Val Tyr Thr Thr Tyr Cys Pro Ala Gln Tyr Thr Ile Tyr Glu Pro
   1745 1750 1755
   Val Ile Arg Leu Lys Gly Gln Met Lys Thr Gln Leu Ser Gln Arg
   1760 1765 1770
   Pro Phe Ser Ser Lys Glu Val Gln Ser Ile Leu Leu Glu Pro His
   1775 1780 1785
   His Leu  Lys Asn Leu Gln Pro  Thr Glu Tyr Lys Thr  Ile Gln Gly
       1790                 1795                 1800
   Ile Leu  His Glu Ile Gly Gly  Thr Gly Ile Phe Val  Phe Leu Phe
       1805                 1810                 1815
   Ala Arg  Val Val Glu Leu Ser  Ser Cys Glu Glu Thr  Gln Ala Leu
       1820                 1825                 1830
   Ala Leu  Arg Val Ile Leu Ser  Leu Ile Lys Tyr Asn  Gln Gln Arg
       1835                 1840                 1845
   Val His  Glu Leu Glu Asn Cys  Asn Gly Leu Ser Met  Ile His Gln
       1850                 1855                 1860
   Val Leu  Ile Lys Gln Lys Cys  Ile Val Gly Phe Tyr  Ile Leu Lys
       1865                 1870                 1875
   Thr Leu  Leu Glu Gly Cys Cys  Gly Glu Asp Ile Ile  Tyr Met Asn
       1880                 1885                 1890
   Glu Asn  Gly Glu Phe Lys Leu  Asp Val Asp Ser Asn  Ala Ile Ile
       1895                 1900                 1905
   Gln Asp  Val Lys Leu Leu Glu  Glu Leu Leu Leu Asp  Trp Lys Ile
       1910                 1915                 1920
   Trp Ser  Lys Ala Glu Gln Gly  Val Trp Glu Thr Leu  Leu Ala Ala
       1925                 1930                 1935
   Leu Glu  Val Leu Ile Arg Ala  Asp His His Gln Gln  Met Phe Asn
       1940                 1945                 1950
   Ile Lys  Gln Leu Leu Lys Ala  Gln Val Val His His  Phe Leu Leu
       1955                 1960                 1965
   Thr Cys  Gln Val Leu Gln Glu  Tyr Lys Glu Gly Gln  Leu Thr Pro
       1970                 1975                 1980
   Met Pro  Arg Glu Val Cys Arg  Ser Phe Val Lys Ile  Ile Ala Glu
       1985                 1990                 1995
   Val Leu  Gly Ser Pro Pro Asp  Leu Glu Leu Leu Thr  Ile Ile Phe
       2000                 2005                 2010
   Asn Phe  Leu Leu Ala Val His  Pro Pro Thr Asn Thr  Tyr Val Cys
       2015                 2020                 2025
   His Asn Pro Thr Asn Phe Tyr Phe Ser Leu His Ile Asp Gly Lys
   2030 2035 2040
   Ile Phe Gln Glu Lys Val Arg Ser Ile Met Tyr Leu Arg His Ser
   2045 2050 2055
   Ser Ser Gly Gly Arg Ser Leu Met Ser Pro Gly Phe Met Val Ile
   2060 2065 2070
   Ser Pro Ser Gly Phe Thr Ala Ser Pro Tyr Glu Gly Glu Asn Ser
   2075 2080 2085
   Ser Asn Ile Ile Pro Gln Gln Met Ala Ala His Met Leu Arg Ser
   2090 2095 2100
   Arg Ser Leu Pro Ala Phe Pro Thr Ser Ser Leu Leu Thr Gln Ser
   2105 2110 2115
   Gln Lys Leu Thr Gly Ser Leu Gly Cys Ser Ile Asp Arg Leu Gln
   2120 2125 2130
   Asn Ile Ala Asp Thr Tyr Val Ala Thr Gln Ser Lys Lys Gln Asn
   2135 2140 2145
   Ser Leu Gly Ser Ser Asp Thr Leu Lys Lys Gly Lys Glu Asp Ala
   2150 2155 2160
   Phe Ile Ser Ser Cys Glu Ser Ala Lys Thr Val Cys Glu Met Glu
   2165 2170 2175
   Ala Val Leu Ser Ala Gln Val Ser Val Ser Asp Val Pro Lys Gly
   2180 2185 2190
   Val Leu Gly Phe Pro Val Val Lys Ala Asp His Lys Gln Leu Gly
   2195 2200 2205
   Ala Glu Pro Arg Ser Glu Asp Asp Ser Pro Gly Asp Glu Ser Cys
   2210 2215 2220
   Pro Arg Arg Pro Asp Tyr Leu Lys Gly Leu Ala Ser Phe Gln Arg
   2225 2230 2235
   Ser His Ser Thr Ile Ala Ser Leu Gly Leu Ala Phe Pro Ser Gln
   2240 2245 2250
   Asn Gly Ser Ala Ala Val Gly Arg Trp Pro Ser Leu Val Asp Arg
   2255 2260 2265
   Asn Thr Asp Asp Trp Glu Asn Phe Ala Tyr Ser Leu Gly Tyr Glu
   2270 2275 2280
   Pro Asn Tyr Asn Arg Thr Ala Ser Ala His Ser Val Thr Glu Asp
   2285 2290 2295
   Cys Leu Val Pro Ile Cys Cys Gly Leu Tyr Glu Leu Leu Ser Gly
   2300 2305 2310
   Val Leu Leu Ile Leu Pro Asp Val Leu Leu Glu Asp Val Met Asp
   2315 2320 2325
   Lys Leu Ile Gln Ala Asp Thr Leu Leu Val Leu Val Asn His Pro
   2330 2335 2340
   Ser Pro Ala Ile Gln Gln Gly Val Ile Lys Leu Leu Asp Ala Tyr
   2345 2350 2355
   Phe Ala Arg Ala Ser Lys Glu Gln Lys Asp Lys Phe Leu Lys Asn
   2360 2365 2370
   Arg Gly Phe Ser Leu Leu Ala Asn Gln Leu Tyr Leu His Arg Gly
   2375 2380 2385
   Thr Gln Glu Leu Leu Glu Cys Phe Ile Glu Met Phe Phe Gly Arg
   2390 2395 2400
   His Ile Gly Leu Asp Glu Glu Phe Asp Leu Glu Asp Val Arg Asn
   2405 2410 2415
   Met Gly Leu Phe Gln Lys Trp Ser Val Ile Pro Ile Leu Gly Leu
   2420 2425 2430
   Ile Glu Thr Ser Leu Tyr Asp Asn Ile Leu Leu His Asn Ala Leu
   2435 2440 2445
   Leu Leu Leu Leu Gln Ile Leu Asn Ser Cys Ser Lys Val Ala Asp
   2450 2455 2460
   Met Leu Leu Asp Asn Gly Leu Leu Tyr Val Leu Cys Asn Thr Val
   2465 2470 2475
   Ala Ala Leu Asn Gly Leu Glu Lys Asn Ile Pro Met Ser Glu Tyr
   2480 2485 2490
   Lys Leu Leu Ala Cys Asp Ile Gln Gln Leu Phe Ile Ala Val Thr
   2495 2500 2505
   Ile His Ala Cys Ser Ser Ser Gly Ser Gln Tyr Phe Arg Val Ile
   2510 2515 2520
   Glu Asp Leu Ile Val Met Leu Gly Tyr Leu Gln Asn Ser Lys Asn
   2525 2530 2535
   Lys Arg Thr Gln Asn Met Ala Val Ala Leu Gln Leu Arg Val Leu
   2540 2545 2550
   Gln Ala Ala Met Glu Phe Ile Arg Thr Thr Ala Asn His Asp Ser
   2555 2560 2565
   Glu Asn Leu Thr Asp Ser Leu Gln Ser Pro Ser Ala Pro His His
   2570 2575 2580
   Ala Val Val Gln Lys Arg Lys Ser Ile Ala Gly Pro Arg Lys Phe
   2585 2590 2595
   Pro Leu Ala Gln Thr Glu Ser Leu Leu Met Lys Met Arg Ser Val
   2600 2605 2610
   Ala Asn Asp Glu Leu His Val Met Met Gln Arg Arg Met Ser Gln
   2615 2620 2625
   Glu Asn Pro Ser Gln Ala Thr Glu Thr Glu Leu Ala Gln Arg Leu
   2630 2635 2640
   Gln Arg Leu Thr Val Leu Ala Val Asn Arg Ile Ile Tyr Gln Glu
   2645 2650 2655
   Phe Asn Ser Asp Ile Ile Asp Ile Leu Arg Thr Pro Glu Asn Val
   2660 2665 2670
   Thr Gln Ser Lys Thr Ser Val Phe Gln Thr Glu Ile Ser Glu Glu
   2675 2680 2685
   Asn Ile His His Glu Gln Ser Ser Val Phe Asn Pro Phe Gln Lys
   2690 2695 2700
   Glu Ile Phe Thr Tyr Leu Val Glu Gly Phe Lys Val Ser Ile Gly
   2705 2710 2715
   Ser Ser Lys Ala Ser Gly Ser Lys Gln Gln Trp Thr Lys Ile Leu
   2720 2725 2730
   Trp Ser  Cys Lys Glu Thr Phe  Arg Met Gln Leu Gly  Arg Leu Leu
       2735                 2740                 2745
   Val His  Ile Leu Ser Pro Ala  His Ala Ala Gln Glu  Arg Lys Gln
       2750                 2755                 2760
   Ile Phe  Glu Ile Val His Glu  Pro Asn His Gln Glu  Ile Leu Arg
       2765                 2770                 2775
   Asp Cys  Leu Ser Pro Ser Leu  Gln His Gly Ala Lys  Leu Val Leu
       2780                 2785                 2790
   Tyr Leu  Ser Glu Leu Ile His  Asn His Gln Gly Glu  Leu Thr Glu
       2795                 2800                 2805
   Glu Glu  Leu Gly Thr Ala Glu  Leu Leu Met Asn Ala  Leu Lys Leu
       2810                 2815                 2820
   Cys Gly  His Lys Cys Ile Pro  Pro Ser Ala Ser Thr  Lys Ala Asp
       2825                 2830                 2835
   Leu Ile  Lys Met Ile Lys Glu  Glu Gln Lys Lys Tyr  Glu Thr Glu
       2840                 2845                 2850
   Glu Gly  Val Asn Lys Ala Ala  Trp Gln Lys Thr Val  Asn Asn Asn
       2855                 2860                 2865
   Gln Gln  Ser Leu Phe Gln Arg  Leu Asp Ser Lys Ser  Lys Asp Ile
       2870                 2875                 2880
   Ser Lys  Ile Ala Ala Asp Ile  Thr Gln Ala Val Ser  Leu Ser Gln
       2885                 2890                 2895
   Gly Asn  Glu Arg Lys Lys Val  Ile Gln His Ile Arg  Gly Met Tyr
       2900                 2905                 2910
   Lys Val  Asp Leu Ser Ala Ser  Arg His Trp Gln Glu  Leu Ile Gln
       2915                 2920                 2925
   Gln Leu  Thr His Asp Arg Ala  Val Trp Tyr Asp Pro  Ile Tyr Tyr
       2930                 2935                 2940
   Pro Thr  Ser Trp Gln Leu Asp  Pro Thr Glu Gly Pro  Asn Arg Glu
       2945                 2950                 2955
   Arg Arg  Arg Leu Gln Arg Cys  Tyr Leu Thr Ile Pro  Asn Lys Tyr
       2960                 2965                 2970
   Leu Leu  Arg Asp Arg Gln Lys  Ser Glu Asp Val Val  Lys Pro Pro
       2975                 2980                 2985
   Leu Ser  Tyr Leu Phe Glu Asp  Lys Thr His Ser Ser  Phe Ser Ser
       2990                 2995                 3000
   Thr Val  Lys Asp Lys Ala Ala  Ser Glu Ser Ile Arg  Val Asn Arg
       3005                 3010                 3015
   Arg Cys  Ile Ser Val Ala Pro  Ser Arg Glu Thr Ala  Gly Glu Leu
       3020                 3025                 3030
   Leu Leu  Gly Lys Cys Gly Met  Tyr Phe Val Glu Asp  Asn Ala Ser
       3035                 3040                 3045
   Asp Thr  Val Glu Ser Ser Ser  Leu Gln Gly Glu Leu  Glu Pro Ala
       3050                 3055                 3060
   Ser Phe  Ser Trp Thr Tyr Glu  Glu Ile Lys Glu Val  His Lys Arg
       3065                 3070                 3075
   Trp Trp  Gln Leu Arg Asp Asn  Ala Val Glu Ile Phe  Leu Thr Asn
       3080                 3085                 3090
   Gly Arg  Thr Leu Leu Leu Ala  Phe Asp Asn Thr Lys  Val Arg Asp
       3095                 3100                 3105
   Asp Val  Tyr His Asn Ile Leu  Thr Asn Asn Leu Pro  Asn Leu Leu
       3110                 3115                 3120
   Glu Tyr  Gly Asn Ile Thr Ala  Leu Thr Asn Leu Trp  Tyr Thr Gly
       3125                 3130                 3135
   Gln Ile  Thr Asn Phe Glu Tyr  Leu Thr His Leu Asn  Lys His Ala
       3140                 3145                 3150
   Gly Arg  Ser Phe Asn Asp Leu  Met Gln Tyr Pro Val  Phe Pro Phe
       3155                 3160                 3165
   Ile Leu  Ala Asp Tyr Val Ser  Glu Thr Leu Asp Leu  Asn Asp Leu
       3170                 3175                 3180
   Leu Ile  Tyr Arg Asn Leu Ser  Lys Pro Ile Ala Val  Gln Tyr Lys
       3185                 3190                 3195
   Glu Lys  Glu Asp Arg Tyr Val  Asp Thr Tyr Lys Tyr  Leu Glu Glu
       3200                 3205                 3210
   Glu Tyr  Arg Lys Gly Ala Arg  Glu Asp Asp Pro Met  Pro Pro Val
       3215                 3220                 3225
   Gln Pro  Tyr His Tyr Gly Ser  His Tyr Ser Asn Ser  Gly Thr Val
       3230                 3235                 3240
   Leu His  Phe Leu Val Arg Met  Pro Pro Phe Thr Lys  Met Phe Leu
       3245                 3250                 3255
   Ala Tyr  Gln Asp Gln Ser Phe  Asp Ile Pro Asp Arg  Thr Phe His
       3260                 3265                 3270
   Ser Thr  Asn Thr Thr Trp Arg  Leu Ser Ser Phe Glu  Ser Met Thr
       3275                 3280                 3285
   Asp Val  Lys Glu Leu Ile Pro  Glu Phe Phe Tyr Leu  Pro Glu Phe
       3290                 3295                 3300
   Leu Val  Asn Arg Glu Gly Phe  Asp Phe Gly Val Arg  Gln Asn Gly
       3305                 3310                 3315
   Glu Arg  Val Asn His Val Asn  Leu Pro Pro Trp Ala  Arg Asn Asp
       3320                 3325                 3330
   Pro Arg  Leu Phe Ile Leu Ile  His Arg Gln Ala Leu  Glu Ser Asp
       3335                 3340                 3345
   Tyr Val  Ser Gln Asn Ile Cys  Gln Trp Ile Asp Leu  Val Phe Gly
       3350                 3355                 3360
   Tyr Lys  Gln Lys Gly Lys Ala  Ser Val Gln Ala Ile  Asn Val Phe
       3365                 3370                 3375
   His Pro  Ala Thr Tyr Phe Gly  Met Asp Val Ser Ala  Val Glu Asp
       3380                 3385                 3390
   Pro Val  Gln Arg Arg Ala Leu  Glu Thr Met Ile Lys  Thr Tyr Gly
       3395                 3400                 3405
   Gln Thr  Pro Arg Gln Leu Phe  His Met Ala His Val  Ser Arg Pro
       3410                 3415                 3420
   Gly Ala  Lys Leu Asn Ile Glu  Gly Glu Leu Pro Ala  Ala Val Gly
       3425                 3430                 3435
   Leu Leu  Val Gln Phe Ala Phe  Arg Glu Thr Arg Glu  Gln Val Lys
   3440 3445 3450
   Glu Ile Thr Tyr Pro Ser Pro Leu Ser Trp Ile Lys Gly Leu Lys
   3455 3460 3465
   Trp Gly Glu Tyr Val Gly Ser Pro Ser Ala Pro Val Pro Val Val
   3470 3475 3480
   Cys Phe Ser Gln Pro His Gly Glu Arg Phe Gly Ser Leu Gln Ala
   3485 3490 3495
   Leu Pro Thr Arg Ala Ile Cys Gly Leu Ser Arg Asn Phe Cys Leu
   3500 3505 3510
   Leu Met Thr Tyr Ser Lys Glu Gln Gly Val Arg Ser Met Asn Ser
   3515 3520 3525
   Thr Asp Ile Gln Trp Ser Ala Ile Leu Ser Trp Gly Tyr Ala Asp
   3530 3535 3540
   Asn Ile Leu Arg Leu Lys Ser Lys Gln Ser Glu Pro Pro Val Asn
   3545 3550 3555
   Phe Ile Gln Ser Ser Gln Gln Tyr Gln Val Thr Ser Cys Ala Trp
   3560 3565 3570
   Val Pro Asp Ser Cys Gln Leu Phe Thr Gly Ser Lys Cys Gly Val
   3575 3580 3585
   Ile Thr Ala Tyr Thr Asn Arg Phe Thr Ser Ser Thr Pro Ser Glu
   3590 3595 3600
   Ile Glu Met Glu Thr Gln Ile His Leu Tyr Gly His Thr Glu Glu
   3605 3610 3615
   Ile Thr Ser Leu Phe Val Cys Lys Pro Tyr Ser Ile Leu Ile Ser
   3620 3625 3630
   Val Ser Arg Asp Gly Thr Cys Ile Ile Trp Asp Leu Asn Arg Leu
   3635 3640 3645
   Cys Tyr Val Gln Ser Leu Ala Gly His Lys Ser Pro Val Thr Ala
   3650 3655 3660
   Val Ser Ala Ser Glu Thr Ser Gly Asp Ile Ala Thr Val Cys Asp
   3665 3670 3675
   Ser Ala  Gly Gly Gly Ser Asp  Leu Arg Leu Trp Thr  Val Asn Gly
       3680                 3685                 3690
   Asp Leu  Val Gly His Val His  Cys Arg Glu Ile Ile  Cys Ser Val
       3695                 3700                 3705
   Ala Phe  Ser Asn Gln Pro Glu  Gly Val Ser Ile Asn  Val Ile Ala
       3710                 3715                 3720
   Gly Gly  Leu Glu Asn Gly Ile  Val Arg Leu Trp Ser  Thr Trp Asp
       3725                 3730                 3735
   Leu Lys  Pro Val Arg Glu Ile  Thr Phe Pro Lys Ser  Asn Lys Pro
       3740                 3745                 3750
   Ile Ile  Ser Leu Thr Phe Ser  Cys Asp Gly His His  Leu Tyr Thr
       3755                 3760                 3765
   Ala Asn  Ser Asp Gly Thr Val  Ile Ala Trp Cys Arg  Lys Asp Gln
       3770                 3775                 3780
   Gln Arg  Leu Lys Gln Pro Met  Phe Tyr Ser Phe Leu  Ser Ser Tyr
       3785                 3790                 3795
   Ala Ala  Gly
       3800

## Description

### CROSS-REFERENCE TO RELATED APPLICATIONS

### REFERENCE TO SEQUENCE LISTING

### FIELD OF THE INVENTION

The field of the invention is generally related to compositions, devices, and methods for enhancing vascular tissue regeneration, as well as reducing morbidity and mortality associated with neointimal hyperplasia and fibroproliferative disease.

### BACKGROUND OF THE INVENTION

Tissue repair is a complex, step-wise process that facilitates the ordered removal and replacement of dead or damaged cells in response to stimulatory cytokine signaling from local pro-inflammatory cells at the site of injury (Wynn and Baron, Semin Liver Dis., 30(3):245-257 (2010) ; Wynn, Clin. Invest. 117:524-529 (2007)). However, when tissue-repair responses are inappropriate or persist for an extended period, for example, in response to chronic disease or repeated injury, the normal regenerative mechanisms can give rise to pathological states resulting from excessive neotissue growth and accumulation of extracellular matrix (ECM) components at the site of injury.

Excessive or repeated activation of inflammatory and pro-coagulant mechanisms at the site of vascular injury is thought to contribute significantly to the development of intimal hyperplasia. Under pathological conditions, vascular injury causes denudation of the endothelial layer and triggers a series of acute and chronic inflammatory responses characterized by production of various growth factors and inflammatory cytokines (Murakami, et al., Am JPhysiol Lung Cell Mol Physiol., 272:L197-L202 (1997); Cotran, et al., J Am Soc Nephrol., 1:225-235 (1990)). Intimal hyperplasia occurring after surgical procedures such as angioplasty and stenting, surgical bypasses or endarterectomy for coronary and/or peripheral arterial disease can cause restenosis of blood vessels in up to 80% of patients (Seedial, et al., J Vasc Surg., 57(5): 1403-1414 (2013); Glagov, Circulation, 89:2888-2891 (1994)). It has been shown that neointima formation can be accelerated by the presence of foreign material such as prostheses within blood vessels, and uncontrolled neointimal hyperplasia is a major obstacle limiting the long-term clinical efficiency of cardiovascular intervention (Frank, et al., Curr. Opin. Lipidol., 15:523 (2004)). In addition, neointima formation is associated with the development and progression of several proliferative cardiovascular diseases, including atherosclerosis, hypertension, and diabetic vascular complications.

Regeneration of the endothelial layer inhibits the development of neointima and facilitates vascular repair (Bauters, et al., Prog. Cardiovasc. Dis., 40:107-116 (1997); Kinlay, et al., Curr. Opin. Lipidol., 12:383 (2001)). However, currently available anti-neointimal drugs indiscriminately block the proliferation of both endothelial cells (EC) and smooth muscle cells (SMC) resulting in impaired re-endothelialization and prolonging the wound healing process. There remains a need to develop an anti-proliferation strategy that is SMC-sensitive.

Unregulated inflammatory responses to repeated tissue damage can also give rise to pathological fibrosis of tissue and organs such as the skin, liver, heart and lung (Huang and Ogawa, Connect Tissue Res., 53(3):187-196 (2012)). Chronic damage and inflammation of liver tissue leads to the accumulation of ECM proteins that cause excessive scar formation and distort the hepatic architecture, which in turn leads to cirrhosis and liver failure (Battler and Brenner, J Clin Invest., 115:209-218 (2005)). Likewise, excessive deposition of fibrotic tissue within the lung gives rise to progressive scarring and irreversible destruction of lung architecture that ultimately leads to organ malfunction, disruption of gas exchange, and death from respiratory failure.

Activated platelets increase leukocyte adhesion to the damaged endothelium and promote leukocyte activation through deposition of chemokines on the endothelium. This enables leukocytes to firmly attach to the vessel wall and transmigrate into the subendothelial tissue. However, the platelet-derived chemokines are also known to play a role in neointimal proliferation, restenosis after arterial injury and atherosclerosis (Chandrasekar, et al., JAm College Cardiology, Vol 35, No. 3, pp. 555-562 (2000)). Certain patient groups having abnormal platelet function, including those with renal disease, diabetes mellitus and hyperlipidemia, are also at increased risk for restenosis.

Fibroproliferative disorders are a leading cause of morbidity and mortality in the United States (Bitterman and Henke, Chest, 99(3):s81-s84 (1991)). In particular, hepatic and pulmonary fibrotic diseases are typically refractory to treatment and carry a high mortality rate. Established liver cirrhosis has a 10-year mortality rate of 34-66%, and is responsible for approximately 27,000 deaths each year in the United States alone. The prevalence of idiopathic pulmonary fibrosis (IPF) is estimated at between 14 and 42.7 per 100,000, and this figure has been rising decade after decade for the last 30 years (Olson, et al., Am JRespir Crit Care Med. 176:277-2843 (2007); Gribbin, et al., Thorax, 61:980-985 (2006)). Currently, the most effective treatment for hepatic or pulmonary fibrosis is organ transplantation.

Therefore, it is an object of the invention to provide compositions, devices, grafts, and methods of use thereof for modulating immune processes to reduce or prevent neointima formation, stenosis, restenosis or a combination thereof in a subject.

It is also an object of the invention to provide compositions, devices, grafts, and methods of use thereof to reduce or prevent inappropriate or deleterious platelet activation in a subject.

It is also an object of the invention to provide compositions, methods, and devices for reducing or preventing the immune response to an implantable prosthesis in a subject.

It is a further object of the invention to provide compositions, methods, and devices to promote healing and inhibit scar formation, adhesions and fibroproliferative diseases in a subject.

WO 99/51741 discloses complexes of the LYST or LYST-2 protein with proteins identified as interacting with LYST or LYST-2 (14-3-3 protein, HS1 protein, Hrs, BMK1, KB07, Efs, XAP-4, OS9, casein kinase II beta SU, calmodulin, troponin I, importin beta, Fte-1, hERR1, imogen 38, atrophin-1, norbin, HBF-G2, DGS-I, GBDR1, OPA containing protein, M4 protein, LIP1, LIP2, LIP3, LIP4, LIP5, LIP6, LIP7, LIP8, LIP9 and LIP10, and derivatives, fragments and analogs thereof). WO 99/51741 also discloses nucleic acids encoding the LIP1, LIP2, LIP3, LIP4, LIP5, LIP6, LIP7, LIP8, LIP9, and LIP10 protein, or derivatives, fragments and analogs thereof. Methods of screening the complexes or proteins for efficacy in treating and/or preventing certain diseases and disorders, particularly atopic diseases, autoimmune diseases, neurodegenerative disease, cancer, pigmentation disorders, platelet dysfunction and viral diseases, are also disclosed therein.

### SUMMARY OF THE INVENTION

It has been established that inhibition of the lysosomal trafficking regulator ("LYST") protein is associated with reduced inflammatory responses at the site of tissue damage. Compositions and methods for inhibition of the expression and function of the LYST protein are described. The compositions and methods can be useful for the modulation of immune processes that contribute to formation of neointima and fibroproliferative disorders by altering macrophage, platelet and natural killer cell function to create a pro-regenerative immune response.

Accordingly, the invention relates to a pharmaceutical composition for use in a method of reducing macrophage infiltration, scar formation, or stenosis at a site of graft implantation in a subject comprising:
a) one or more LYST inhibitors in an amount effective to
   reduce macrophage infiltration, natural killer cell activation, and/or platelet activation in the subject, and
b) a physiologically acceptable carrier,
wherein the composition is administered to the subject prior to surgery, at the time of surgery, and/or within days after surgery, and wherein the one or more LYST inhibitors is in an amount between 0.1-1000 mg/kg body weight of a human and does not prevent wound healing.

Some further particular embodiments of the invention are defined in the appended claims.

In one embodiment one or more LYST inhibitors is an antibody.

In another embodiment one or more LYST inhibitors is a functional nucleic acid. Exemplary functional nucleic acids include an antisense molecule, siRNA, miRNA, aptamers, ribozymes, triplex forming molecules, RNAi, and external guide sequences. The one or more functional nucleic acids can be expressed from an expression vector.

In some embodiments the compositions of one or more LYST inhibitors include a delivery vehicle. Exemplary delivery vehicles include nanoparticles, microparticles, micelles, emulsions, synthetic lipoprotein particles, liposomes, carbon nanotubes, gels, and coatings. The disclosed compositions can also include one or more additional therapeutic agents. Vascular grafts and medical devices including one or more LYST inhibitors are disclosed. In some embodiments the composition of one or more LYST inhibitors is coated onto or incorporated into the graft or device. Exemplary medical devices include stents, implants, needles, cannulas, catheters, shunts, balloons, and valves. The medical device can be a stent, such as a drug eluting stent that elutes the composition of one or more LYST inhibitors. Exemplary vascular grafts include autologous, preserved autologous, allogeneic, xenogenic or synthetic grafts.

A pharmaceutical composition as defined by Claim 1 for use in a method of reducing the formation of scar tissue in a subject, by administering to a subject in need thereof said composition including one or more LYST inhibitors are also provided. A pharmaceutical composition as defined by Claim 1 for use in a methods of reducing platelet activation and venous thrombosis or arterial thrombosis in a subject include administering to a subject in need thereof the composition including one or more LYST inhibitors is also provided. In some embodiments the subject is at risk of developing, or has developed, restenosis or other vascular proliferation disorders. In further embodiments the subject has undergone, is undergoing, or will undergo vascular trauma, angioplasty, vascular surgery, or transplantation arteriopathy. The use of the pharmaceutical composition in said methods can promote healing, reduce the development of hypertrophic scarring, keloids, or adhesions, reduce fibrosis of the liver, fibrosis of the lungs, fibrosis of the heart or fibrosis of the kidneys, reduces neointima formation, stenosis or restenosis, or any combination thereof in the subject relative to an untreated control subject. The use of the pharmaceutical composition in said methods can promote integration but block encapsulation of one or more prosthetic devices such as pacemakers, nerve stimulators, pacemaker leads, replacement heart valves and artificial joints or c components thereof. The use of the pharmaceutical composition in said methods can be effective to treat or prevent neointima formation at a site of implantation of a vascular implant, a site of vascular injury, or a site of surgery in the subject, relative to an untreated control subject. The methods can be effective to reduce or prevent the expression of platelet derived growth factor, transforming growth factor beta, or any combination thereof in a subject relative to an untreated control subject.

### BRIEF DESCRIPTION OF THE DRAWINGS

Figure 1 is a histogram showing the diameter (mm) of the conduit within biodegradable conduit grafts two weeks after implant into beige mice (bg; black graph) and wild type mice (WT; grey graph). N=10 and N=25 for beige mice and WT mice, respectively.
Figure 2 is a histogram showing the number of macrophage cells (cell/HPF) within biodegradable conduit grafts explanted from beige mice (Bg; grey graph) and wild type mice (WT; black graph), respectively, two weeks after implant. P = 0.0048.

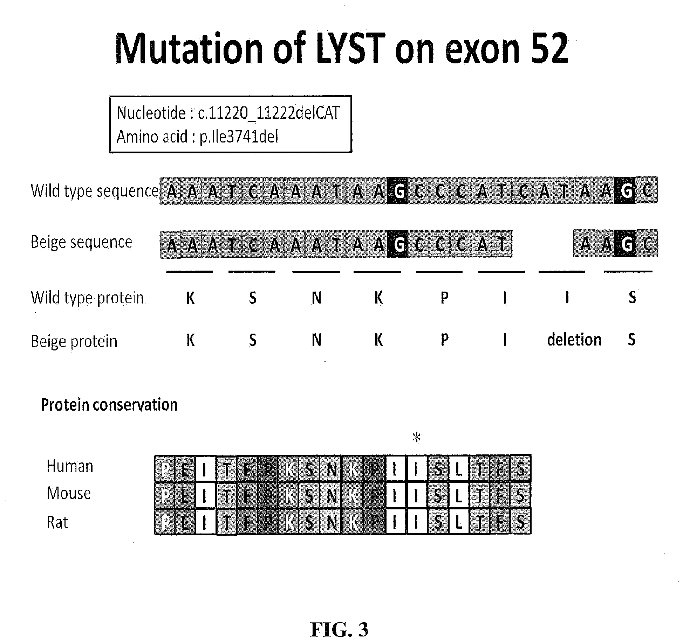

Figure 3 is a schematic showing the difference between the nucleotide sequences (at top) of the wild type (WT) and Beige (Bg) mice in the LYST gene on exon 52. Corresponding differences between the amino acid sequences of the LYST protein of WT and Bg mice are also shown in an amino acid sequence alignment (middle). The amino acid sequence alignment (at bottom) shows conservation amongst the amino acid sequences of LYST polypeptides from human, mouse and rat. The position of the deletion within the LYST of Beige (Bg) mice is indicated by *. Nucleic acids and amino acids are represented using the respective standard single-letter codes.
Figure 4 is a histogram showing the relative level of mRNA corresponding to the LYST gene product, amplified using each of 5 different oligonucleotide primers (LYST-1, LYST-3, LYST-4, LYST-5 and LYST-6), in cells obtained from wild-type mice (WT-1(1); WT-2 (2)), Beige mice (Beige-1 (3); Beige-2 (4)) or a standard "Raw" cell line (5), respectively.
Figure 5 is a histogram showing the incidence of stenosis (%) in C57BL/6 (wild type) mice and C57BL/6 Lyst^{tmlb} knockout mice, respectively, (n=5) after 2-week implantation . ***p = 0.0001.
Figure 6 is a histogram showing the amount of platelet derived growth factor (PDGF) secreted from PRP (µg/ml) in resting (control) and thrombin activated C57BL/6 (wild type) mice, as well as C57BL/6 Beige (Bg) mice, respectively. Total concentrations of secreted PDGF from each group (n=7) were compared to resting, non-activated controls (n=5).

### DETAILED DESCRIPTION OF THE INVENTION

### I. Definitions

The term "stenosis" refers to an abnormal narrowing in a blood vessel that occurs following an injury to the vessel wall (endothelium). In some embodiments, stenosis involves a reduction in the circumference of a lumen of 50% or more. The term "restenosis" refers to stenosis at a previously stenotic site or narrowing of the lumen of a blood vessel or synthetic graft following an interventional procedure. Restenosis, as used herein, encompasses occlusion. Exemplary injuries that result in stenosis or restenosis include trauma to an atherosclerotic lesion (as seen with angioplasty or stent), a resection of a lesion (as seen with endarterectomy), an external trauma (*e.g.*, a cross-clamping injury), or a surgical anastomosis.

The term "neointimal stenosis" refers to abnormal narrowing in a blood vessel resulting from neointimal formation.

The term "neointima" refers to a renewed or thickened layer of intima (inner lining) formed in a blood vessel in response to signals from injured endothelial cells.

The terms "scar tissue" and "scarring" refer to the fibrous tissue that is produced to replace damaged tissue following injury.

The terms "fibroproliferative disorders", "FPD" and "fibrotic diseases are used interchangeably and include benign and malignant diseases or conditions that result from the abnormal and excessive deposition of connective tissue.

The term "Platelet Activation" is the step-wise physiological process that gives rise to adherence and aggregation of circulating platelets in response to tissue injury, such as damage or interruption of the endothelium. Platelet activation gives rise to expression and secretion of chemotactic agents such as platelet derived growth factor (PDGF) and transforming growth factor beta (TGFβ).

"Pharmaceutically acceptable carrier" encompasses any of the standard pharmaceutical carriers, such as a phosphate buffered saline solution, water and emulsions such as an oil/water or water/oil emulsion, and various types of wetting agents.

"Inhibit" or other forms of the word such as "inhibiting" or "inhibition" means to hinder or restrain a particular characteristic. It is understood that this is typically in relation to some standard or expected value, *i.e.*, it is relative, but that it is not always necessary for the standard or relative value to be referred to. For example, "inhibits LYST" means hindering, interfering with or restraining the activity of the LYST gene relative to a standard or a control. "Inhibits LYST" can also mean to hinder or restrain the synthesis, expression or function of the LYST protein relative to a standard or control.

"Treatment" or "treating" means to administer a composition to a subject or a system with an undesired condition (e.g., restenosis or a fibroproliferative disorder). The condition can include a disease. "Prevention" or "preventing" means to administer a composition to a subject or a system at risk for the condition. The condition can be a predisposition to a disease. The effect of the administration of the composition to the subject (either treating and/or preventing) can be, but is not limited to, the cessation of a particular symptom of a condition, a reduction or prevention of the symptoms of a condition, a reduction in the severity of the condition, the complete ablation of the condition, a stabilization or delay of the development or progression of a particular event or characteristic, or minimization of the chances that a particular event or characteristic will occur.

As used herein, the term "antibody" is used in the broadest sense unless clearly indicated otherwise. Therefore, an "antibody" can be naturally occurring or man-made, such as monoclonal antibodies produced by conventional hybridoma technology. Antibodies include monoclonal and polyclonal antibodies as well as fragments containing the antigen-binding domain and/or one or more complementarity determining regions of these antibodies. "Antibody" refers to any form of antibody or antigen binding fragment thereof and includes monoclonal antibodies (including full length monoclonal antibodies), polyclonal antibodies, multi-specific antibodies (e.g., bi-specific antibodies), and antibody fragments.

### II. Compositions

It has been established that the processes of tissue repair can be mediated by manipulation of the expression and function of the LYST gene. Specifically, the loss of function of the LYST gene product gives rise to abnormal function in certain immune cells, resulting in immune dis-function and an associated reduction in proliferative disorders. Immune cells that can be influenced by modulation of the LYST gene or LYST protein include innate immune cells, macrophages and platelets.

### Innate Immune Cells

Innate immune processes including inflammation, removal of damaged cells and debris, as well as the development of neotissue and other wound repair mechanisms are an important component of the physiological responses to injury. Tissue repair has four distinct stages, including: a) clotting/coagulation; b) inflammation; c) fibroblast migration/proliferation; and d) a final remodeling phase where normal tissue architecture is restored. In the earliest stages after tissue damage, epithelial cells and/or endothelial cells release inflammatory mediators that initiate an antifibrinolytic-coagulation cascade that triggers clotting and development of a provisional extracellular matrix (ECM). Aggregation and subsequent degranulation of platelets promotes blood vessel dilation and increased permeability, allowing efficient recruitment of inflammatory cells such as neutrophils, macrophages, lymphocytes, and eosinophils to the damaged tissue. Neutrophils are the most abundant inflammatory cell at the earliest stages of wound healing, but are quickly replaced by macrophages after neutrophil degranulation. Activated macrophages and neutrophils debride the wound, eliminate any invading organisms and produce a variety of cytokines and chemokines that amplify the inflammatory response as well as trigger fibroblast proliferation and recruitment. Upon activation, fibroblasts transform into myofibroblasts that secrete α-smooth muscle actin and ECM components. Finally, in the remodeling phase epithelial/endothelial cells divide and migrate over the temporary matrix to regenerate the damaged tissue. Thus, healing and neotissue generation is a finely regulated process that balances the need to regenerate tissue and thicken blood vessel walls, without excessive thickening and stenosis or fibrosis.

### Macrophages

It has been shown that the presence of circulating monocytes and infiltrating macrophages is critical for wound healing and neotissue development (Arras, et al., J Clin Invest, 101(1): 40-50 (1998)). However, the extent of macrophage infiltration at a site of tissue damage has also been correlated with proliferative dysregulation and neointima formation (Hibino, et al., FASEB J. 25(12):4253-63 (2011)). Further, numerous studies have indicated that macrophages and fibroblasts are the main effector cells involved in the pathogenesis of fibrosis (reviewed in Wynn, Nat Rev Immunol. 4(8):583-94 (2004)).

Following vascular damage, inflammatory monocyte cells (CD16-hi, CD64-hi and CD14-hi in humans; CD115+, CD11b+ and Ly6c-hi in mice) are recruited to the damaged tissue and differentiate into activated macrophages (Emr1-hi in humans; F4/80-hi in mice) upon exposure to local growth factors, pro-inflammatory cytokines and microbial compounds (Geissmann et al., Science 327: 656-661 (2010)). Excessive macrophage infiltration results in stenosis, whilst complete inhibition of macrophage infiltration prevents neotissue formation (Hibino, et al., FASEB J. 25(12):4253-63 (2011).

Two distinct states of polarized activation for macrophages have been defined: the classically activated (M1) macrophage phenotype and the alternatively activated (M2) macrophage phenotype (Gordon and Taylor, Nat. Rev. Immunol. 5: 953-964 (2005); Mantovani et al., Trends Immunol. 23: 549-555 (2002)). The role of the classically activated (M1) macrophage is an effector cell in TH1 cellular immune responses, whereas the alternatively activated (M2) macrophage appears to be involved in immunosuppression and wound healing/tissue repair. M1 and M2 macrophages have distinct chemokine and chemokine receptor profiles, with M1 secreting the TH1 cell-attracting chemokines CXCL9 and CXCL10, and with M2 macrophages expressing chemokines CCL17, CCL22 and CCL24.

The presence of M2 macrophages has been associated with neointima development and stenosis (Hibino, et al., FASEB J. 25(12):4253-63 (2011)). The correlation between the extent of macrophage infiltration, neotissue formation and stenosis at certain time points following tissue graft implantation provides means to prevent stenosis through modulation of macrophage activity.

Further, macrophages are typically located close to collagen-producing myofibroblast cells, and it has been shown that monocyte-derived macrophages critically perpetuate inflammatory responses after injury as a prerequisite for fibrosis (Wynn and Barron, Semin Liver Dis., 30(3):245-257 (2010)). Macrophages produce pro-fibrotic mediators that activate fibroblasts, including platelet-derived growth factor (PDGF), a potent chemotactic agent, and transforming growth factor beta (TGF-B). Specifically, a marked increase of the non-classical M2 (CD14+, CD16+) subset of macrophages has been correlated with pro-inflammatory cytokines and clinical progression in patients suffering from chronic liver disease. During fibrosis progression, monocyte-derived macrophages release cytokines perpetuating chronic inflammation as well as directly activate hepatic stellate cells (HSCs), resulting in their proliferation and trans-differentiation into collagen-producing myofibroblasts (Zimmermann, et al., PLOS One, 5(6):e11049 (2010)).

### Platelets

Aggregated platelets assist the repair of blood vessels by secreting chemicals that attract fibroblasts from surrounding connective tissue into the wounded area to heal the wound or, in the case of dysregulated inflammatory responses, form scar tissue. In response to tissue injury, platelets become activated and release a multitude of growth factors which stimulate the deposition of extracellular matrix, such as platelet-derived growth factor (PDGF), a potent chemotactic agent, as well as transforming growth factor beta (TGF-β). Both of these growth factors have been shown to play a significant role in the repair and regeneration of connective tissues. PDGF functions as a primary mitogen and chemo-attractant which significantly augments the influx of fibroblasts and inflammatory cells, as well as stimulating cell proliferation and gene expression. PDGF enables leukocytes to firmly attach to the vessel wall and finally to transmigrate into the subendothelial tissue. However, the platelet-derived chemokines are also known to induce smooth muscle cell (SMC) proliferation and play a role in neointimal proliferation and organ fibrosis (Chandrasekar, et al., JAm College Cardiology, Vol 35, No. 3, pp. 555-562 (2000)). Increased expression of PDGF and its receptors is associated with scleroderma lung and skin tissue. Specifically, there is evidence for an autocrine PDGF-receptor mediated signaling loop in scleroderma lung and skin fibroblasts, implicating both TGF-β and PDGF pathways in chronic fibrosis in scleroderma (Trojanowska, Rheumatology;47:v2-v4 (2008)). In addition, deregulation of PDGF signaling is associated with cardiovascular indications such as pulmonary hypertension, and atherosclerosis.

Media layer smooth muscle cell (SMC) proliferation and migration in response to injury-induced PDGF are essential events contributing to neointimal thickening (Fingerle, et al., Proc Natl Acad Sci., 86:8412 (1989); Clowes, et al., Circ. Res., 56:139-145 (1985)) which eventually leads to blood vessel narrowing and stenosis.

Other healing-associated growth factors released by platelets include basic fibroblast growth factor, insulin-like growth factor 1, platelet-derived epidermal growth factor, and vascular endothelial growth factor.

Thus, inhibitors of the LYST gene and LYST protein can create a pro-regenerative immune environment that enhances wound healing and prevents proliferative disorders such as intimal hyperplasia, development of excessive scar tissue and fibrotic disease. Modulation of LYST can also modulate platelet activity and function. Thus, inhibitors of the LYST gene and/or LYST protein can be used to reduce the biological functions of platelets, such as platelet aggregation and the production/expression of platelet derived growth factor (PDGF).

Compositions for promoting tissue regeneration and preventing or reducing diseases characterized by the formation of excessive scar tissue by blockade of expression and/or function of the LYST protein are disclosed.

### A. LYST

The lysosomal trafficking regulator (LYST) gene product is an ubiquitous protein associated with the transport of intracellular material to the lysosome. It has been established that mutations which reduce or inhibit the function of the LYST protein interfere with the normal functions of macrophages and also impact the biological activities of natural killer (NK) cells and platelets. It is believed that modulation of the LYST protein provides a means to modulate immune processes that give rise to vascular proliferative disorders, neointima formation, fibrosis and excessive scarring.

### 1. The LYST Gene

The human LYST gene is located at chromosome 1, (segment 1q42.1-q42.2; base pairs 235,661,030 to 235,883,707) (Barrat, et al., Am. J. Hum. Genet, 59:625-632 (1996)). Nucleic acid sequences for the LYST gene product are known in the art. See, for example, NCBI Reference Sequence: NM_000081.3, Homo sapiens lysosomal trafficking regulator (LYST), transcript variant 1, mRNA, which provides the nucleic acid sequence: (SEQ ID NO: 1). Nucleotide sequences that have at least 80%, 85%, 90%, 95%, 99% or 100% amino acid sequence identity to SEQ ID NO: 1 are also disclosed.

### 2. The LYST Protein

The LYST polypeptide is a 3,801 amino acid cytoplasmic protein (also known as lysosomal trafficking regulator, CHS protein, CHS1 or LYST protein) with a molecular weight of approximately 429 kDa (Barbosa, et al., Nature, 382(6588): 262-265 (1996)).
The LYST protein exists as one of three isoforms and is predicted to adopt a helical structure (Barbosa, et al., Nature, 382(6588): 262-265 (1996)).

The LYST protein is highly conserved across mammalian species and is expressed at low levels in all cell types, but is abundantly expressed in adult and fetal thymus, peripheral blood leukocytes, bone marrow and several regions of adult brain (see review by Dotta, et al., Orphanet Journal of Rare Diseases, 8:168 (2013), and references therein).

Amino acid sequences of the human LYST protein are known in the art. See, for example, GenBank Accession No. U67615:

LYST polypeptides that have at least 80%, 85%, 90%, 95%, 99% or 100% amino acid sequence identity to SEQ ID NO: 2 are disclosed.

Although the LYST protein has been associated with many diverse cellular activities, particularly in intracellular protein trafficking in endosomes and lysosomes, the biological function of the LYST protein has remained largely unknown.

Abnormal expression and function of the LYST gene product has been implicated in numerous pathological disorders, including autoimmune diseases, hyperproliferative disorders and platelet dysfunction. Specifically, mutations in the LYST gene are associated with the human disease Chediak-Higashi syndrome (CHS) (Barrat, et al., Am. J. Hum. Genet, 59:625-632 (1996)). In the CHS patient, the LYST gene contains a frame-shift mutation at nucleotides 117-118 of the coding domain.

CHS is a rare lysosomal storage disorder characterized by hypopigmentation, severe immunologic deficiency, a bleeding tendency, neurologic abnormalities, abnormal intracellular transport to and from the lysosome and giant inclusion bodies in a variety of cell types. At least 30 mutations in the LYST gene have been identified in people with Chediak-Higashi syndrome. These mutations impair the normal function of the lysosomal trafficking regulator protein, which disrupts the size, structure, and function of Iysosomes and related structures within cells. People with LYST mutations have abnormally large lysosomes and related structures in cells throughout the body. These enlarged structures interfere with normal cell functions. Enlarged lysosomes in immune system cells, such as macrophages, prevent these cells from responding appropriately to bacteria and other foreign invaders; large perinuclear lysosomes are arranged in a tubular fashion within the macrophage cells of persons with CHS. Affected neutrophils and monocytes have a chemotactic and migratory capacity that is about 40% that of normal cells. Affected patients also have a tendency to bleeding due to platelet dysfunction. As a result of decreased responsiveness to chemotactic stimuli, the malfunctioning immune system cannot protect the body from severe, recurrent infections. CHS is often fatal because patients are immunocompromised and are incapable of mounting an effective immune response to infection.

In the Beige (Bg) mouse strain the mouse homologue of LYST (*"Lyst"*) is disrupted by a deletion that precludes the expression of functional *Lyst* protein. Bg mice were found to exhibit reduced proliferative diseases, including a lower rate of stenosis following implantation of vascular tissue grafts, as compared to wild-type mice. The effect of the dysfunctional *Lyst* protein in Bg mice is highly macrophage-specific, as determined by bone marrow transplant experiments (see Examples).

### C. Inhibitors of LYST

It has been established that blockade of the LYST protein can reduce or prevent immune processes that give rise to vascular proliferative disorders, fibroproliferative disease and excessive scar tissue following injury. Immuno-modulatory agents that inhibit or reduce the transcription, translation or function of the LYST protein are disclosed.

Inhibitors of LYST can bind to the LYST gene or to LYST protein and directly or indirectly block the biological function of LYST protein. Inhibitors can also block the biological function of one or more signaling pathways that constitute the down-stream biological function of LYST. In some embodiments, inhibitors of LYST act by preventing endogenous ligands of the LYST protein from interacting with or binding directly to the LYST protein. The inhibitors can block protein-protein interactions involving the LYST protein, or they can prevent or reduce the functional activity of a complex of the LYST protein and a ligand. Inhibitors that bind directly to the LYST protein may act by direct occlusion of an active site on the LYST protein, or through indirect occlusion, such as by stearic blockade of LYST interactions. For example, in some embodiments the inhibitor obstructs or occludes the function of a protein interaction domain, such as the solenoid protein domain formed by tandem copies of the WD40 repeat motif. In other embodiments, inhibitors bind to a location that is spatially distinct from an active site.

Inhibitors that bind to the LYST protein can prevent LYST function by mechanisms including, but not limited to, inducing dimerization, inducing oligomerization, inducing conformational changes, preventing catalytic functions, inducing degradation, inducing uptake by immune cells, preventing uptake by target cells, preventing ligand binding, preventing phosphorylation, inducing denaturation, preventing one or more post-translational modifications or otherwise altering the native tertiary structure of the LYST protein.

It is understood that initiation or transduction of cellular signaling pathways by LYST can require binding of a ligand to the LYST protein. Therefore, proteins, antibodies or small molecules that block signal transduction pathways involving LYST and optionally prevent co-ligation of LYST and its receptors are useful immune-modulatory agents. Classes of LYST inhibitors discussed below include antibodies and functional nucleic acids.

### 1. Antibodies

Antibodies that inhibit the function of LYST by binding directly to the LYST protein, its ligands or its accessory molecules are disclosed. Any specific antibody can be used in the methods and compositions provided herein. Antibodies can include an antigen binding site that binds to an epitope on the LYST protein. Binding of an antibody to LYST can inhibit or reduce the function of the LYST protein *via* one or more distinct mechanisms.

In some embodiments, the antibody or antigen binding fragment binds specifically to an epitope within the protein encoded by the amino acid sequence of SEQ ID NO: 2. The epitope can be a linear epitope and can include one or more consecutive amino acids of the primary sequence of SEQ ID NO: 2. In other embodiments, the antibody or antigen binding fragment thereof can bind a conformational epitope that includes a 3-D surface feature, shape, or tertiary structure of the LYST protein. In some embodiments, a 3-D surface feature can include any number of amino acids from SEQ ID NO: 2, or the corresponding residues in a homolog, ortholog, paralog, or variant thereof.

In some embodiments, the antibody or antigen binding fragment that binds specifically to an epitope within the protein encoded by the amino acid sequence of SEQ ID NO: 2 can only bind if the protein encoded by the amino acid sequence of SEQ ID NO: 2 is not bound by a ligand or small molecule.

Various types of antibodies and antibody fragments can be used in the disclosed compositions and methods, including whole immunoglobulin of any class, fragments thereof, and synthetic proteins containing at least the antigen binding variable domain of an antibody. The antibody can be an IgG antibody, such as IgG₁, IgG₂, IgG₃, or IgG₄. An antibody can be in the form of an antigen binding fragment including a Fab fragment, F(ab')2 fragment, a single chain variable region, and the like. Antibodies can be polyclonal or monoclonal (mAb). Monoclonal antibodies include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they specifically bind the target antigen and/or exhibit the desired biological activity (U.S. Patent No. 4,816,567; and Morrison, et al., Proc. Natl. Acad. Sci. USA, 81: 6851-6855 (1984)). The disclosed antibodies can also be modified by recombinant means, for example by deletions, additions or substitutions of amino acids, to increase efficacy of the antibody in mediating the desired function. Substitutions can be conservative substitutions. For example, at least one amino acid in the constant region of the antibody can be replaced with a different residue (see, *e.g.*, U.S. Patent No. 5,624,821; U.S. Patent No. 6,194,551; WO 9958572; and Angal, et al., Mol. Immunol. 30:105-08 (1993)). In some cases changes are made to reduce undesired activities, e.g., complement-dependent cytotoxicity. The antibody can be a bi-specific antibody having binding specificities for at least two different antigenic epitopes. In one embodiment, the epitopes are from the same antigen. In another embodiment, the epitopes are from two different antigens. Bi-specific antibodies can include bi-specific antibody fragments (see, *e.g.*, Hollinger, et al., Proc. Natl. Acad. Sci. U.S.A., 90:6444-48 (1993); Gruber, et al., J. Immunol., 152:5368 (1994)).

A variety of antibodies that bind the human LYST protein are commercially available from multiple sources, for example, Santa Cruz Biotechnology, CA, USA, Cat. No. sc-136746). Antibodies can be generated by any means known in the art. Exemplary descriptions means for antibody generation and production include Delves, Antibody Production: Essential Techniques (Wiley, 1997); Shephard, et al., Monoclonal Antibodies (Oxford University Press, 2000); Goding, Monoclonal Antibodies: Principles And Practice (Academic Press, 1993); and Current Protocols In Immunology (John Wiley & Sons, most recent edition). Fragments of intact Ig molecules can be generated using methods well known in the art, including enzymatic digestion and recombinant means.

### 2. Functional Nucleic Acids

Functional nucleic acids that inhibit the transcription, translation or function of LYST gene products are disclosed. Functional nucleic acids are nucleic acid molecules that have a specific function, such as binding a target molecule or catalyzing a specific reaction. As discussed in more detail below, functional nucleic acid molecules can be divided into the following non-limiting categories: antisense molecules, siRNA, miRNA, aptamers, ribozymes, triplex forming molecules, RNAi, and external guide sequences. The functional nucleic acid molecules can act as effectors, inhibitors, modulators, and stimulators of a specific activity possessed by a target molecule, or the functional nucleic acid molecules can possess a *de novo* activity independent of any other molecules.

Functional nucleic acid molecules can interact with any macromolecule, such as DNA, RNA, polypeptides, or carbohydrate chains. Thus, functional nucleic acids can interact with the mRNA or the genomic DNA of the LYST polypeptide or they can interact with the LYST polypeptide itself. Functional nucleic acids are often designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place. Therefore the disclosed compositions can include one or more functional nucleic acids designed to reduce expression or function of the LYST protein.

In some embodiments, the composition includes a functional nucleic acid or polypeptide designed to target and reduce or inhibit expression or translation of LYST mRNA; or to reduce or inhibit expression, reduce activity, or increase degradation of LYST protein. In some embodiments, the composition includes a vector suitable for *in vivo* expression of the functional nucleic acid.

In some embodiments, a functional nucleic acid or polypeptide is designed to target a segment of the nucleic acid encoding the amino acid sequence of SEQ ID NO: 2, or the complement thereof, or variants thereof having a nucleic acid sequence 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identical to a nucleic acid encoding the amino acid sequence of SEQ ID NO: 2.

In other embodiments, a functional nucleic acid or polypeptide is designed to target a segment of the nucleic acid sequence of SEQ ID NO: 1, or the complement thereof, or variants thereof having a nucleic acid sequence at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identical to SEQ ID NO: 1
In some embodiments, the function nucleic acid hybridizes to the nucleic acid of SEQ ID NO: 1, or a complement thereof, for example, under stringent conditions. In some embodiments, the function nucleic acid hybridizes to a nucleic acid sequence that encodes SEQ ID NO: 2, or a complement thereof, for example, under stringent conditions.

Methods of making and using vectors for *in vivo* expression of the disclosed functional nucleic acids such as antisense oligonucleotides, siRNA, shRNA, miRNA, EGSs, ribozymes, and aptamers are known in the art.

### i. Antisense Molecules

The functional nucleic acids can be antisense molecules. Antisense molecules are designed to interact with a target nucleic acid molecule through either canonical or non-canonical base pairing. The interaction of the antisense molecule and the target molecule is designed to promote the destruction of the target molecule through, for example, RNAse H mediated RNA-DNA hybrid degradation. Alternatively the antisense molecule is designed to interrupt a processing function that normally would take place on the target molecule, such as transcription or replication. Antisense molecules can be designed based on the sequence of the target molecule. There are numerous methods for optimization of antisense efficiency by finding the most accessible regions of the target molecule. Exemplary methods include *in vitro* selection experiments and DNA modification studies using DMS and DEPC. It is preferred that antisense molecules bind the LYST target molecule with a dissociation constant (K_{d}) less than or equal to 10⁻⁶, 10⁻⁸, 10⁻¹⁰, or 10⁻¹².

### ii. Aptamers

The functional nucleic acids can be aptamers. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP and theophiline, as well as large molecules, such as reverse transcriptase and thrombin. Aptamers can bind very tightly with K_{d}'s from the target molecule of less than 10⁻¹² M. It is preferred that the aptamers bind the LYST target molecule with a K_{d} less than 10⁻⁶, 10⁻⁸, 10⁻¹⁰, or 10⁻¹². Aptamers can bind the target molecule with a very high degree of specificity. For example, aptamers have been isolated that have greater than a 10,000 fold difference in binding affinities between the target molecule and another molecule that differ at only a single position on the molecule. It is preferred that the aptamer have a K_{d} with the LYST target molecule at least 10, 100, 1000, 10,000, or 100,000 fold lower than the K_{d} with a background binding molecule. It is preferred when doing the comparison for a molecule such as a polypeptide, that the background molecule be a different polypeptide.

### iii. Ribozymes

The functional nucleic acids can be ribozymes. Ribozymes are nucleic acid molecules that are capable of catalyzing a chemical reaction, either intra-molecularly or inter-molecularly. It is preferred that the ribozymes catalyze intermolecular reactions. Different types of ribozymes that catalyze nuclease or nucleic acid polymerase-type reactions which are based on ribozymes found in natural systems, such as hammerhead ribozymes are disclosed. Ribozymes that are not found in natural systems, but which have been engineered to catalyze specific reactions *de novo* are also disclosed. Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for targeting specific cleavage of nucleic acids because recognition of the target substrate is based on the target substrates sequence.

### iv. Triplex Forming Oligonucleotides

The functional nucleic acids can be triplex forming oligonucleotide molecules. Triplex forming functional nucleic acid molecules are molecules that can interact with either double-stranded or single-stranded nucleic acid. When triplex molecules interact with a target region, a structure called a triplex is formed in which there are three strands of DNA forming a complex dependent on both Watson-Crick and Hoogsteen base-pairing. Triplex molecules are preferred because they can bind target regions with high affinity and specificity. It is preferred that the triplex forming molecules bind the target molecule with a K_{d} less than 10⁻⁶, 10⁻⁸, 10⁻¹⁰, or 10⁻¹².

### v. External Guide Sequences

The functional nucleic acids can be external guide sequences. External guide sequences (EGSs) are molecules that bind a target nucleic acid molecule forming a complex, which is recognized by RNase P, which then cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. Similarly, eukaryotic EGS/RNAse P-directed cleavage of RNA can be utilized to cleave desired targets within eukaryotic cells. Representative examples of how to make and use EGS molecules to facilitate cleavage of a variety of different target molecules are known in the art.

### vi. RNA Interference

In some embodiments, the functional nucleic acids induce gene silencing through RNA interference (siRNA). Expression of the LYST gene can be effectively silenced in a highly specific manner through RNA interference.

Gene silencing was originally observed with the addition of double stranded RNA (dsRNA) (Fire, et al. (1998) Nature, 391:806-11; Napoli, et al. (1990) Plant Cell 2:279-89; Hannon, (2002) Nature, 418:244-51). Once dsRNA enters a cell, it is cleaved by an RNase III-like enzyme called Dicer, into double stranded small interfering RNAs (siRNA) 21-23 nucleotides in length that contain 2 nucleotide overhangs on the 3' ends (Elbashir, et al., Genes Dev., 15:188-200 (2001); Bernstein, et al., Nature, 409:363-6 (2001); Hammond, et al., Nature, 404:293-6 (2000); Nykanen, et al., Cell, 107:309-21 (2001); Martinez, et al., Cell, 110:563-74 (2002)). The effect of iRNA or siRNA or their use is not limited to any type of mechanism.

In one embodiment, a siRNA triggers the specific degradation of homologous LYST RNA molecules, such as LYST mRNAs, within the region of sequence identity between both the siRNA and the target LYST RNA.

Sequence specific gene silencing can be achieved in mammalian cells using synthetic, short double-stranded RNAs that mimic the siRNAs produced by the enzyme dicer (Elbashir, et al., Nature, 411:494-498 (2001)) (Ui-Tei, et al., FEBS Lett, 479:79-82 (2000)).

siRNA can be chemically or *in* vitro-synthesized or can be the result of short double-stranded hairpin-like RNAs (shRNAs) that are processed into siRNAs inside the cell. For example, WO 02/44321 discloses siRNAs capable of sequence-specific degradation of target mRNAs when base-paired with 3' overhanging ends, and describes the method of making these siRNAs. Synthetic siRNAs are generally designed using algorithms and a conventional DNA/RNA synthesizer. Suppliers include Ambion (Austin, Texas), ChemGenes (Ashland, Massachusetts), Dharmacon (Lafayette, Colorado), Glen Research (Sterling, Virginia), MWB Biotech (Esbersberg, Germany), Proligo (Boulder, Colorado), and Qiagen (Vento, The Netherlands). siRNA can also be synthesized *in vitro* using kits such as Ambion's SILENCER® siRNA Construction Kit. In some embodiments, the composition includes a vector expressing the functional nucleic acid. The production of siRNA from a vector is more commonly done through the transcription of a short hairpin RNAse (shRNAs). Kits for the production of vectors including shRNA are available, such as, for example, Imgenex's GENESUPPRESSOR™ Construction Kits and Invitrogen's BLOCK-IT™ inducible RNAi plasmid and lentivirus vectors. In some embodiments, the functional nucleic acid is siRNA, shRNA, or miRNA.

### B. Excipients, Delivery Vehicles and Devices

LYST inhibitors can be administered and taken up into the cells of a subject with or without the aid of a delivery vehicle. Appropriate delivery vehicles for the disclosed inhibitors are known in the art and can be selected to suit the particular inhibitor. In a preferred embodiment, the inhibitor is delivered by injection intravenously, subcutaneously, intraperitoneally, or locally. Typical carriers are saline, phosphate buffered saline, and other injectable carriers.

Formulations including one or more LYST inhibitors with or without delivery vehicles are disclosed. The disclosed LYST inhibitors can be formulated into pharmaceutical compositions including one or more pharmaceutically acceptable carriers. Pharmaceutical compositions can be formulated for different mechanisms of administration, according to the inhibitor and the intended use. Pharmaceutical compositions formulated for administration by parenteral (intramuscular, intraperitoneal, intravenous (IV) or subcutaneous injection), topical or transdermal (either passively or using iontophoresis or electroporation) routes of administration or using bioerodible inserts are disclosed.

### 1. Parenteral Administration

In some embodiments, one or more LYST inhibitors and optionally a delivery vehicle are formulated for administration in an aqueous solution, by parenteral injection. The formulation may also be in the form of a suspension or emulsion. In general, pharmaceutical compositions are provided including effective amounts of an active agent, targeting moiety, and optional a delivery vehicle and optionally include pharmaceutically acceptable diluents, preservatives, solubilizers, emulsifiers, adjuvants and/or carriers. Such compositions include the diluents sterile water, buffered saline of various buffer content (*e.*g., Tris-HCl, acetate, phosphate), pH and ionic strength and optionally additives such as detergents and solubilizing agents (*e.*g., TWEEN® 20, TWEEN® 80 also referred to as polysorbate 20 or 80), anti-oxidants (*e.g*., ascorbic acid, sodium metabisulfite), and preservatives (*e.g.*, Thimersol, benzyl alcohol) and bulking substances (*e.g.*, lactose, mannitol). Examples of non-aqueous solvents or vehicles are propylene glycol, polyethylene glycol, vegetable oils, such as olive oil and corn oil, gelatin, and injectable organic esters such as ethyl oleate. The formulations may be lyophilized and redissolved/resuspended immediately before use. The formulation may be sterilized by, for example, filtration through a bacteria retaining filter, by incorporating sterilizing agents into the compositions, by irradiating the compositions, or by heating the compositions.

### 2. Pulmonary and Mucosal Administration

In further embodiments, one or more LYST inhibitors and optionally a delivery vehicle are formulated for administration to the mucosa, such as the mouth, eyes, lungs, nasal, oral (sublingual, buccal), vaginal, or rectal mucosa.

Formulations for administration to the mucosa will typically be spray dried drug particles, which may be incorporated into a tablet, gel, capsule, suspension or emulsion. Standard pharmaceutical excipients are available from any formulator.

In one embodiment, the compounds are formulated for pulmonary delivery, such as intranasal administration or oral inhalation. The respiratory tract is the structure involved in the exchange of gases between the atmosphere and the blood stream. The upper and lower airways are called the conducting airways. The terminal bronchioli divide into respiratory bronchiole, which then lead to the ultimate respiratory zone, the alveoli, or deep lung. The deep lung, or alveoli, is the primary target of inhaled therapeutic aerosols for systemic drug delivery. Therapeutic agents that are active in the lungs can be administered systemically and targeted *via* pulmonary absorption. The term aerosol as used herein refers to any preparation of a fine mist of particles, which can be in solution or a suspension, whether or not it is produced using a propellant. Aerosols can be produced using standard techniques, such as ultra-sonication or highpressure treatment.

Carriers for pulmonary formulations can be divided into those for dry powder formulations and for administration as solutions. Aerosols for the delivery of therapeutic agents to the respiratory tract are known in the art. For administration via the upper respiratory tract, the formulation can be formulated into a solution, e.g., water or isotonic saline, buffered or un-buffered, or as a suspension, for intranasal administration as drops or as a spray. Preferably, such solutions or suspensions are isotonic relative to nasal secretions and of about the same pH, ranging e.g., from about pH 4.0 to about pH 7.4 or, from pH 6.0 to pH 7.0. Buffers should be physiologically compatible and include, simply by way of example, phosphate buffers. One skilled in the art can readily determine a suitable saline content and pH for an innocuous aqueous solution for nasal and/or upper respiratory administration.

Compositions can be delivered to the lungs while inhaling and traverse across the lung epithelial lining to the blood stream when delivered either as an aerosol or spray dried particles having an aerodynamic diameter of less than about 5 microns.

Dry powder formulations ("DPFs") with large particle size have improved flowability characteristics, such as less aggregation, easier aerosolization, and potentially less phagocytosis. Dry powder aerosols for inhalation therapy are generally produced with mean diameters primarily in the range of less than 5 microns, although a preferred range is between one and ten microns in aerodynamic diameter. Large "carrier" particles (containing no drug) have been co-delivered with therapeutic aerosols to aid in achieving efficient aerosolization among other possible benefits. Formulations for pulmonary delivery include unilamellar phospholipid vesicles, liposomes, or lipoprotein particles. Formulations and methods of making such formulations containing nucleic acid are well known to one of ordinary skill in the art. A wide range of mechanical devices designed for pulmonary delivery of therapeutic products can be used, including but not limited to nebulizers, metered dose inhalers, and powder inhalers, all of which are familiar to those skilled in the art.

### 3. Topical and Transdermal Administration

The active agent and optional delivery vehicle can be applied topically. Topical administration can include application directly to the vasculature or to tissues or prostheses, for example during surgery, or by direct administration to the skin.

Transdermal formulations may also be prepared. These will typically be ointments, lotions, sprays, or patches, all of which can be prepared using standard technology. Transdermal formulations can include penetration enhancers. Standard pharmaceutical excipients for topical or transdermal administration are available from any formulator.

### 4. Controlled Delivery Matrices

Controlled release polymeric devices can be made for long term release systemically following implantation of a polymeric device (rod, cylinder, film, disk) or injection (microparticles). The matrix can be in the form of microparticles such as microspheres, where LYST inhibitors are dispersed within a solid polymeric matrix or microcapsules, where the core is of a different material than the polymeric shell, and one or more LYST inhibitor(s) is dispersed or suspended in the core, which may be liquid or solid in nature. Unless specifically defined herein, microparticles, microspheres, and microcapsules are used interchangeably. Alternatively, the polymer may be cast as a thin slab or film, ranging from nanometers to four centimeters, a powder produced by grinding or other standard techniques, or even a gel such as a hydrogel.

Either non-biodegradable or biodegradable matrices can be used for delivery of disclosed inhibitors of LYST, although biodegradable matrices are preferred. These may be natural or synthetic polymers, although synthetic polymers are preferred due to the better characterization of degradation and release profiles. The polymer is selected based on the period over which release is desired. In some cases linear release may be most useful, although in others a pulse release or "bulk release" may provide more effective results. The polymer may be in the form of a hydrogel (typically in absorbing up to about 90% by weight of water), and can optionally be cross-linked with multivalent ions or polymers.

The matrices can be formed by solvent evaporation, spray drying, solvent extraction and other methods known to those skilled in the art. Bioerodible microspheres can be prepared using any of the methods developed for making microspheres for drug delivery, for example, as described by Mathiowitz and Langer, J. Controlled Release 5:13-22 (1987); Mathiowitz, et al., Reactive Polymers 6:275-283 (1987); and Mathiowitz, et al., J. Appl. Polymer Sci. 35:755-774 (1988). The devices can be formulated for local release to treat the area of implantation or injection, which will typically deliver a dosage that is much less than the dosage for treatment of an entire body. The devices can also be formulated for local or systemic delivery.

Micro and nanoparticles designed to deliver cargo such as drugs and antibodies to the vasculature, to vascular smooth muscle cells, or sites or clots or thrombosis are known in the art. See, for example, Wickline, et al., Arteriosclerosis, Thrombosis, and Vascular Biology, 26:435-441 (2006), published online (Dec. 2005), and U.S. Published Application Nos. 2002/0168320, 2003/0086867, 2003/0129136, 2004/0058951, 2004/0115192, 2006/0147380, 2006/0239919, 2007/0140965, 2007/0202040, 2007/0258908, 2008/0175792, 2008/0247943, and 2013/0064765.

For example, perfluorocarbon nanoparticles, previously considered as artificial blood substitutes, have been developed into a platform technology for molecular imaging and targeted drug delivery, *i.e.*, a so-called "theranostic" technology. These lipid-encapsulated particles, which are nominally 250 nm in diameter, can be administered intravenously and are typically constrained by size to the intact vasculature.

In some embodiments the delivery vehicle is a liposome. If the LYST inhibitor is an antibody, protein or small molecule, the preferred delivery vehicle can be a liposome. Liposomes are disclosed for the delivery of the disclosed LYST inhibitors directly to a certain cell type, for example macrophage cells. Macrophage cells can internalize liposomes, leading to the delivery of the one or more inhibitors to the intracellular compartments of the macrophage. Suitable methods, materials and lipids for making liposomes are known in the art. Liposome delivery vehicles are commercially available from multiple sources. Liposomes can be formed from a single lipid bilayer (*i.e.*, the liposome can be unilamellar) or several concentric lipid bilayers (*i.e.*, the liposome can be multilamellar). The liposome may be formed from a single lipid; however, in some embodiments, the liposome is formed from a combination of more than one lipid. The lipids can be neutral, anionic or cationic at physiologic pH.

Suitable neutral and anionic lipids include sterols and lipids such as cholesterol, phospholipids, lysolipids, lysophospholipids, and sphingolipids. Neutral and anionic lipids include, but are not limited to, phosphatidylcholine (PC) (such as egg PC, soy PC), including 1,2-diacyl-glycero-3-phosphocholines; phosphatidylserine (PS), phosphatidylglycerol, phosphatidylinositol (PI); glycolipids; sphingophospholipids, such as sphingomyelin, sphingoglycolipids (also known as 1-ceramidyl glucosides), such as ceramide galactopyranoside, gangliosides and cerebrosides; fatty acids, sterols containing a carboxylic acid group such as cholesterol or derivatives thereof; and 1 ,2-diacyl-sn-glycero-3-phosphoethanolamines, including 1,2-dioleoyl-sn-Glycero-3-phosphoethanolamine or 1,2-dioleolylglyceryl phosphatidylethanolamine (DOPE), 1 ,2-dihexadecylphosphoethanolamine (DHPE), 1,2-distearoylphosphatidylcholine (DSPC), 1,2-dipalmitoylphosphatidylcholine (DPPC), and 1,2-dimyristoylphosphatidylcholine (DMPC). Trimethyl ammonium salts, also referred to as TAP lipids, for example as a methylsulfate salt. Suitable TAP lipids include, but are not limited to, DOTAP (dioleoyl-), DMTAP (dimyristoyl-), DPTAP (dipalmitoyl-), and DSTAP (distearoyl-). Other suitable cationic lipids include dimethyldioctadecyl ammonium bromide (DDAB), 1 ,2-diacyloxy-3-trimethylammonium propanes, N-[1-(2,3-dioloyloxy)propyl]-N,N-dimethyl amine (DODAP), Other suitable lipids include PEGylated derivatives of the neutral, anionic, and cationic lipids described above. Incorporation of one or more PEGylated lipid derivatives can result in a liposome which displays polyethylene glycol chains on its surface. The resulting liposomes may possess increased stability and circulation time *in vivo* as compared to liposomes lacking PEG chains on their surfaces.

If the LYST inhibitor is a nucleic acid or vector, the delivery vehicle can be a viral vector, for example, a commercially available preparation, such as an adenovirus vector (Quantum Biotechnologies, Inc. (Laval, Quebec, Canada). The viral vector delivery can be *via* a viral system, such as a retroviral vector system which can package a recombinant retroviral genome (see *e.g.*, Pastan et al., (1988) Proc. Natl. Acad. Sci. U.S.A. 85:4486; Miller et al., (1986) Mol. Cell. Biol. 6:2895). The recombinant retrovirus can then be used to infect and thereby deliver to the infected cells nucleic acid encoding the LYST inhibitor. The exact method of introducing the altered nucleic acid into mammalian cells is, of course, not limited to the use of retroviral vectors. Other techniques are widely available for this procedure including the use of adenoviral vectors (Mitani et al., Hum. Gene Ther. 5:941-948 (1994)), adeno-associated viral (AAV) vectors (Goodman et al., Blood 84:1492-1500 (1994)), lentiviral vectors (Naidini et al., Science 272:263-267 (1996)), pseudotyped retroviral vectors (Agrawal et al., Exper. Hematol. 24:738-747 (1996)).

Physical transduction techniques can also be used, such as liposome delivery and receptor-mediated and other endocytosis mechanisms (see, for example, Schwartzenberger et al., Blood 87:472-478 (1996)). Commercially available liposome preparations such as LIPOFECTIN, LIPOFECTAMINE (GIBCO-BRL, Inc., Gaithersburg, Md.), SUPERFECT (Qiagen, Inc. Hilden, Germany) and TRANSFECTAM (Promega Biotec, Inc., Madison, Wis.), as well as other liposomes developed according to procedures standard in the art are well known. In addition, the disclosed nucleic acid or vector can be delivered *in vivo* by electroporation, the technology for which is available from Genetronics, Inc. (San Diego, Calif.) as well as by means of a sonoporation or sonication machine (ImaRx Pharmaceutical Corp., Tucson, Ariz.). The disclosed compositions and methods can be used in conjunction with any of these or other commonly used gene transfer methods.

### 5. Grafts and Medical Devices

The disclosed compositions for immunomodulation by inhibition of LYST can be coated onto or incorporated into medical devices, or can be used to pre-treat implantable vascular grafts *ex vivo.*

### Grafts

LYST inhibitors can be used to pre-treat vascular grafts *ex vivo* prior to implantation in a subject. Compositions including one or more LYST inhibitors and optionally a delivery vehicle can be applied to the tissue by methods to insure that they adhere and are distributed throughout the tissue or graft in optimal locations for drug treatment. In some embodiments, one or more LYST inhibitor(s) is delivered using nanoparticles, microparticles, liposomes, micelles, emulsions, gels or coatings that are designed to adhere to the regions of interest, to carry sufficient drug load to provide local treatment for prolonged periods, to release the LYST inhibitors at a desired rate or schedule, to penetrate into tissue or graft to an optimal extent, or a combination thereof.

Grafts for use in surgery can also be formed by seeding cells *ex-vivo* onto a biodegradable scaffold. The grafts can be autologous, for example, saphenous vein or radial artery; preserved autologous, for example, cryopreserved vein; allogeneic; xenogenic; or synthetic, for example, woven polyester, polyurethane (LYCRA®), polytetraflouroethylene (PTFE), GORE-TEX®, or polyethelene terephthalate (DACRON®), Arterial homografts using internal mammary, radial or hypogastric arteries are examples of useful and durable vascular conduits. Exemplary grafts are discussed below.

### Tissue Engineering Vascular Graft

Tissue engineering vascular grafts (TEVG) including one or more inhibitors of LYST are disclosed. Tissue-engineered vascular grafts (TEVGs) hold great promise for advancing the field of congenital heart surgery, where their growth capacity can be used to its fullest potential. For example, TEVGs can be designed for use as a vascular conduit connecting the inferior vena cava to the pulmonary artery in patients undergoing modified Fontan surgery. Long-term (>10 years) results of a pilot study evaluating the use of the TEVG in 25 patients with single ventricle cardiac anomalies demonstrated no graft related deaths or graft failures. It also confirmed the growth capacity of the TEVG making it the first man made graft with growth capacity. The primary graft-related complication was stenosis, effecting approximately 30% of patients with 16% (4/25) requiring angioplasty to treat critical stenosis.

In one embodiment, a TEVG is formed from a biodegradable tubular scaffold fabricated from a polyglycolic acid-fiber tube. The tube can be coated with a copolymer such as a 50:50 ratio of poly lactic acid (PLA) and poly-caprolactone. The scaffold can be seeded with cells prior to implanting into a subject. In some embodiments the cells are autologous cells from the intended recipient.

It has been established that excessive infiltration of macrophages to tissue engineered vascular grafts (TEVGs) promoted scarring, resulting in vessel thickening, occlusion and stenosis. Therefore, the compositions and methods described herein can be used to deliver one or more LYST inhibitors locally and in a controlled fashion to a TEVG to prevent or inhibit infiltration by macrophages.

### Bypass Graft

Bypass grafts including one or more inhibitors of LYST are disclosed. A common form of bypass surgery involves resecting the saphenous vein from the leg for auto-transplantation to the coronary artery. In a significant number of cases these grafts fail, largely due to restenosis caused by neointimal hyperplasia. The compositions and methods described herein can be used to deliver one or more LYST inhibitors locally and in a controlled fashion, to the autologous graft. The inhibitor can be administered prior to, at the time of and/or immediately following surgery. After resection of the saphenous vein the tissue can be (and is often) for hours suspended in saline during chest opening and preparation for graft implantation. Compositions including one or more LYST inhibitors and optional a targeting signal, delivery vehicle or combination thereof can be incubated with the saphenous vein during this time period.

### Arteriovenous Graft

Arteriovenous grafts including one or more inhibitors of LYST are disclosed. End stage renal disease is increasing in the United States. Morbidity of hemodialysis access remains a major quality of life issue for patients; it also represents a significant cost to society. A native arteriovenous fistula (AVF) remains the conduit of choice to provide access for hemodialysis and provides superior results when compared with other options such as a prosthetic AVG. Unfortunately each individual is limited in the number of native AVF that can be created due to the limited number of suitable sites and vessels. Access sites are limited as patients with end stage renal disease usually have severe comorbidity, requiring extensive venipuncture for diagnosis and therapy for life. In patients who have exhausted all options for primary AVF, new access sites must use AV grafts. These grafts are susceptible to restenosis by neointimal hyperplasia limiting their effectiveness. Incubation of a composition including one or more LYST inhibitors, and optionally including a targeting signal, a delivery vehicle, or a combination thereof onto the graft can be done at the time of surgery.

### Medical Devices

In some embodiments, the composition including one or more LYST inhibitors is coated onto, or incorporated into, a medical device or component thereof (such as into polymeric reservoirs in vascular stents) to reduce or inhibit vascular proliferation disorders such as neointima formation in a subject. The device can be a device that is inserted into the subject transiently, or a device that is implanted permanently. In some embodiments, the device is a surgical device.

Examples of medical devices include, but are not limited to, needles, cannulas, catheters, shunts, balloons, and implants such as stents and valves.

In some embodiments, the LYST inhibitor or pharmaceutical composition can be formulated to permit its incorporation onto the medical device, which can apply the inhibitor directly to the site to prevent or treat conditions such restenosis or other vascular proliferation disorders. The LYST inhibitor or pharmaceutical composition thereof can be formulated by including it within a coating on the medical device. There are various coatings that can be utilized such as, for example, polymer coatings that can release the inhibitor over a prescribed time period. The inhibitor, or a pharmaceutical composition thereof, can be embedded directly within the medical device. In some embodiments, the LYST inhibitor is coated onto or within the device in a delivery vehicle such as a microparticle or liposome that facilitates its release and delivery.

### Stents

In some embodiments, the medical device is a vascular implant such as a stent. Stents are utilized in medicine to prevent or eliminate vascular restrictions. Stents can be inserted into a restricted vessel so that the restricted vessel is widened. The experience with such vascular implants indicates that excessive growth of the adjacent cells results again in a restriction of the vessel particularly at the ends of the implants which results in reduced effectiveness of the implants. If a stent is inserted into a human artery for the elimination of an arteriosclerotic stenosis, intimal hyperplasia can occur within a year at the ends of the vascular implant and results in renewed stenosis.

Accordingly, in some embodiments, the stents are coated or loaded with a composition including a LYST inhibitor and optionally a delivery vehicle.

Many stents are commercially available or otherwise know in the art. Stents can be formed, *i.e.*, etched or cut, from a thin tube of suitable material, or from a thin plate of suitable material and rolled into a tube.

Suitable materials for the stent include but are not limited to stainless steel, iridium, platinum, gold, tungsten, tantalum, palladium, silver, niobium, zirconium, aluminum, copper, indium, ruthenium, molybdenum, niobium, tin, cobalt, nickel, zinc, iron, gallium, manganese, chromium, titanium, aluminum, vanadium, and carbon, as well as combinations, alloys, and/or laminations thereof. For example, the stent may be formed from a cobalt alloy, such as L605 or MP35N®, Nitinol (nickel-titanium shape memory alloy), ABI (palladium-silver alloy), Elgiloy® (cobalt-chromium-nickel alloy), *etc.* It is also contemplated that the stent may be formed from two or more materials that are laminated together, such as tantalum that is laminated with MP35N®. The stents may also be formed from wires having concentric layers of different metals, alloys, or other materials. Embodiments of the stent may also be formed from hollow tubes, or tubes that have been filled with other materials. The aforementioned materials and laminations are intended to be examples and are not intended to be limiting in any way.

Stents can be drug-eluting stents. Various drug eluting stents that simultaneously deliver a therapeutic substance to the treatment site while providing artificial radial support to the wall tissue are known in the art. Endo-luminal devices including stents can be coated on their outer surfaces with a substance such as a drug releasing agent, growth factor, antibody, or the like. Stents have also been developed having a hollow tubular structure with holes or ports cut through the sidewall to allow drug elution from a central lumen. Although the hollow nature of the stent allows the central lumen to be loaded with a drug solution that is delivered via the ports or holes in the sidewall of the stent, the hollow tubular structure may not have suitable mechanical strength to provide adequate scaffolding in the vessel.

Stents that elute LYST inhibitors are disclosed. In some embodiments, the devices are also coated or impregnated with a LYST inhibitor and one or more additional therapeutic agents, including, but not limited to, anti-platelet agents, anticoagulant agents, antimicrobial agents, and anti-metabolic agents.

Exemplary stents that can be used with the compositions and methods disclosed herein include, but are not limited to, those described in U.S. Patent Nos. 5,891,108, 6,918,929, 6,923,828, 6,945,992, 6,986,785, 7,060,090, 7,144,419, 7,163,555, 7,323,008, 7,651,527, 7,655,034, 7,678,141, 7,744,645, 7,942,917, 8,001,925, 8,001,925, 8,034,099, 8,048,149, 8,066,760, 8,100,960, 8,157,855, 8,172,893, 8,182,524, 8,187,284, 8,187,322, 8,197,528, 8,206,432, 8,221,490, 8,231,669, 8,236,044, 8,252,048, 8,252,065, 8,257,425, 8,257,431, 8,292,945, 8,298,278, 8,298,280, 8,348,991, 8,348,992, 8,348,993, 8,353,952, 8,359,998, 8,361,140, 8,372,134, 8,372,138, 8,377,112, 8,388,676, 8,398,695, 8,414,637, 8,414,639, and 8,414,656.

### BioProstheses

Multiple other prosthetic devices can include one or more inhibitors of LYST, including but not limited to heart valves, artificial joints, pacemakers, indwelling catheters and cosmetic implants.

It is believed that the disclosed LYST inhibitors can reduce or prevent inflammatory responses and fibroproliferative disorders associated with the presence of a bioprosthesis. Accordingly, in some embodiments, the bioprostheses are coated or loaded with a composition including a LYST inhibitor and optionally a delivery vehicle.

### IV. Uses

Uses of the disclosed LYST inhibitors are provided. The uses can comprise the use of said LYST inhibitors in methods that include administering to a subject an effective amount of a composition including one or more LYST inhibitors to reduce or inhibit the expression or function of LYST in the subject; pretreating a medical device or vascular graft with an effective amount of a composition including a LYST inhibitor to reduce or inhibit neointima formation following insertion or implantation of the device or graft into the subject; or a combination thereof.

### A. Disorders and Diseases to be Treated

LYST inhibitors can be used to alter immune functions, including, but not limited to, macrophage, platelet, and natural killer cell function, creating a pro-regenerative immune environment. Methods of using LYST inhibitors including, but not limited to, methods designed to inhibit or block transcription, translation, or function of the LYST protein can be used to modulate the immune response. In some embodiments, the methods promote vascular neotissue formation, inhibit the formation of neointima and improve vascular patency, for example, following vascular surgery. Methods of using LYST inhibitors for preventing intimal and neointimal hyperplasia in both biological and synthetic vascular conduits are provided. The methods can reduce or prevent TEVG stenosis and improve TEVG function.

Additionally, LYST inhibitors can have broader implications for promoting tissue regeneration, improving wound healing, and modulating the foreign body response. Thus, methods of using LYST inhibitors as an adjunct to regenerative medicine applications are also described. In some embodiments, the methods of using LYST inhibitors can promote healing and reduce scar formation, for example, promoting healing and reducing adhesions after abdominal surgery.

The methods can modulate the amount of Platelet Derived Growth Factor (PDGF) produced as a result of tissue injury. Therefore, the methods can treat diseases associated with excessive or undesirable expression of PDGF.

The methods can also reduce or treat diseases resulting from excessive fibrosis, for example fibrotic diseases of the liver, lung or heart. Finally, methods of anti-LYST therapy can result in immune modulation that alters the foreign body reaction and promotes integration but blocks encapsulation of bio-prostheses, thus improving function and longevity of devices such as pacemaker or nerve stimulators or integration of replacement heart valves or artificial joints.

In preferred embodiments, one or more LYST inhibitors are effective to reduce, inhibit, or delay one or more symptoms of a disease, disorder or condition in a subject. The disclosed LYST inhibitors have a wide variety of therapeutic and prophylactic uses, for example, they can be used to treat or reduce vascular proliferative disorders following injury or various surgical procedures, scarring and fibrotic diseases. Methods of using one or more inhibitors of LYST for treating a disease characterized by the presence of excessive scar or fibrotic tissue are described. Methods of using one or more LYST inhibitors for anti-platelet therapy, for example, for treating diseases resulting from aberrant, excessive or otherwise undesirable platelet activity or PDGF signaling are also provided.

### Vascular Proliferative Disorders

In some embodiments, the disclosed LYST inhibitors can be used to treat or reduce vascular proliferative disorders. Examples of such disorders include, but are not limited to, vascular proliferation involved in atherosclerosis, vascular proliferation following intravascular device implantation, vascular proliferation at the site of vascular anastamosis as generally occurs following revascularization procedure or A-V shunting, vascular proliferation following carotid endarterectomy, and transplant vasculopathy.

Methods of treating or reducing vascular proliferative disorders by administration of one or more LYST inhibitors are provided. The methods typically reduce or inhibit the infiltration of macrophage cells, or the conversion of macrophage cells from M1 to M2 phenotype, or both, compared to a control. In some embodiments, the methods reduce or inhibit proliferation of macrophage cells without reducing or inhibiting vascular neotissue development. A subject can have stenosis, restenosis or other vascular proliferation disorders, or be identified as being at risk for restenosis or other vascular proliferation disorders, for example subjects who have undergone, are undergoing, or will undergo a vascular trauma, angioplasty, surgery, or transplantation arteriopathy, *etc.* Diseases, disorders and conditions that can be treated using the disclosed compositions are discussed in more detail below.

### Vascular Injury

In some embodiments, the composition comprising one or more LYST inhibitors can be applied before, at the time of or following a vascular trauma.

Vascular injury triggers a cascade of events that includes endothelial denudation or dysfunction, inflammation, as well as activation and proliferation of vascular smooth muscle cells (VSMC). Multiple growth factors and cytokines are released by dysfunctional endothelial cells, inflammatory cells, platelets and VSMCs. These growth factors and cytokines mediate chemo-attraction, cell migration, proliferation, apoptosis and matrix modulation, and are implicated in a number of vascular proliferative disorders.

Vascular proliferative diseases and disorders can be initiated by mechanical, biochemical or immunological injury to blood vessel walls. Typical vascular trauma include those associated with both blunt and penetrating injuries including, but not limited to, lacerations, puncture wounds, crush injuries, gunshot wounds, knife wounds, occupational injuries, falls, and motor vehicle accidents, as well as medical interventions, such as surgery or angioplasty.

In some embodiments, the subject has undergone, is undergoing, or will undergo a surgery. Surgeries can include invasive, minimally invasive, or percutaneous surgery. For example, in some embodiments the subject is having surgery to treat or repair abdominal aortic aneurysm, carotid stenosis, varicose veins, peripheral arterial occlusive disease, acute limb ischemia, or aortic dissection. Common vascular surgeries include, but are not limited to, open abdominal aortic aneurysm repair, endovascular aneurysm repair (EVAR), carotid endarterectomy, carotid stenting, vein stripping, sclerotherapy and foam sclerotherapy, endo-venous laser treatment, radiofrequency vein ablation, ambulatory phlebectomy, angioplasty with/out stenting, bypass surgery endarterectomy atherectomy, balloon embolectomy, thrombectomy, bypass surgery, open repair, thoracic endovascular aneurysm repair (TEVAR). A surgeon can apply a composition comprising one or more LYST inhibitors to the surgical site at the time of surgery, prior to surgery or following surgery, to enhance the process of wound healing, or to reduce the development of vascular proliferative disorders, such as those that give rise to stenosis or restenosis.

### Intimal Hyperplasia

Intimal hyperplasia is a physiological healing response to injury of the endothelia of blood vessels. Injury to the endothelial layer triggers a series of acute and chronic inflammatory responses that trigger the aggregation of platelets, the deposition of fibrin and attracts leukocytes to the area (Murakami, et al., Am J Physiol., 272:L197-L202 (1997); Cotran, et al., JAm Soc Nephrol., 1:225-235 (1990)). Thus, regenerative processes that give rise to neovessel formation and intimal hyperplasia appear to be immune-mediated phenomena, similar to the proposed role for monocytes-macrophages in other human vascular biological processes, such as vein graft adaptation (Ratliff and Myles, Arch. Pathol. Lab. Med. 113:772-776 (1989); Motwani and Topol, Circulation 97:916-931 (1998)).

### Stenosis

Intimal hyperplasia can lead to thickening of the Tunica intima of a blood vessel, leading to a complication of stenosis of the blood vessel. Activation of inflammatory and pro-coagulant mechanisms is thought to contribute significantly to the initiation and development of stenosis. Over a period of time ranging from a few weeks to months, smooth muscle cells from the medial region of an injured blood vessel relocate to the intimal region. These cells proliferate and deposit extracellular matrix to form a neointima at the site of the injury in a process analogous to scar formation (Fingerle, et al., Proc Natl Acad Sci., 86:8412 (1989); Clowes, et al., Circ. Res., 56:139-145 (1985)). Thus, a robust healing response leads to an internal thickening of the vessel wall (intimal hyperplasia) and eventually reduces the vessel lumen, causing stenosis. The formation of intimal hyperplasia can be accelerated by the presence of foreign material such as prosthesis within the vessel, and can result from endovascular intervention including angioplasty, bypass, and transplantation arteriopathy, *etc.* (Glagov, Circulation, 89:2888-2891 (1994)).

Therefore, methods of using the disclosed LYST inhibitors for reducing intimal hyperplasia and stenosis are provided.

### Restenosis

Methods to reduce restenosis of the coronary vasculature or the peripheral circulatory system are provided. Restenosis of blood vessels is typically due to intimal hyperplasia. A surgical device, such as a stent, may be inserted to open the stenosed vessel, however this is also problematic because the stent itself can stimulate further intimal hyperplasia. In addition, hyperplastic intimal tissue can grow through the interstices of a bare stent and re-stenose the vessel. Whilst covered stents may prevent this from happening, intimal hyperplasia can still occur at the ends of the stent where there is most irritation of the vessel wall. Patients with in-stent restenosis are at risk of serious complications, as stenosis from intimal hyperplasia is often difficult to treat. Unlike soft atheromatous plaques, these stenoses are firm and require prolonged high inflation pressures to dilate with a balloon. The stenoses often recur and repeated dilatation of the vessel leads to repeated intimal injury and perpetuates the intimal healing response.

Accordingly, the disclosed compositions, devices, or grafts can be administered to a subject to reduce or inhibit smooth muscle cell proliferation, migration, and a combination thereof in an amount effective to reduce or inhibit neointima formation and thereby treat or prevent restenosis and other vascular proliferation disorders in the subject. In some embodiments, the patency of grafts and devices can be increased using a composition containing a LYST inhibitor. Therefore, methods for administering a composition containing a LYST inhibitor to devices and grafts or to the subject prior to or after implantation are provided.

### Atherosclerosis

Atherosclerosis involves multiple processes, including inflammation, vascular proliferation and matrix alteration (reviewed in Dzau, et al., Nat Med,. 8(11) (2002)). In atherosclerosis, VSMC give rise to inflammation, the retention of lipoproteins from the blood, as well as the development of a fibrous deposit that constitute a plaque. Inflammation has been shown to mediate all stages of atherosclerosis: in the development of an atheroma plaque, VSMCs produce pro-inflammatory mediators such as monocyte chemo-attractant proteins, and synthesize matrix molecules that give rise to the retention of lipoproteins from the blood; and following the development of a plaque, local inflammatory milieu can induce collagenase expression and inhibit expression of proteolytic inhibitors, rendering the fibrous cap weak and susceptible to rupture. Therefore, in some embodiments, the disclosed LYST inhibitors are used to treat, reduce, or inhibit vascular proliferation disorders in a subject.

### Angioplasty

In some embodiments, the subject has undergone, is undergoing, or will undergo angioplasty. Angioplasty is the technique of mechanically widening narrowed or obstructed arteries, such as those obstructed as a result of atherosclerosis. Generally, angioplasty includes inserting into a subject's vasculature an empty and collapsed balloon on a guide wire, known as a balloon catheter, which is passed into the narrowed locations and then inflated to a fixed size. The balloon forces expansion of the inner white blood cell/clot plaque deposits and the surrounding muscular wall, opening up the blood vessel for improved flow, and the balloon is then deflated and withdrawn. A stent may or may not be inserted at the time of ballooning to ensure the vessel remains open. Angioplasty includes peripheral angioplasty (*i.e.*, blood vessels outside the coronary arteries, such as in the abdomen, or legs), coronary angioplasty, renal artery angioplasty, carotid angioplasty, and cerebral arteries angioplasty.

In some embodiments, the subject has undergone, is undergoing, or will undergo percutaneous transluminal coronary angioplasty (PTCA). The use of PTCA has greatly reduced the number of fatalities in patients who suffer myocardial infarction (Fischman, et al., N Engl JMed., 331:496-501 (1994); Elezi, et al., Circulation 98:1875-1880 (1998); Bennett and O'Sullivan, Pharmacol Ther., 91:149-166 (2001)). During PTCA, the artery walls are expanded by several times their original diameter in an attempt to increase lumen diameter and improve flow. Unfortunately, this technique is plagued by a high incidence of vessel re-narrowing or restenosis, occurring in 30-40% of patients within 6 months of the procedure (Anderson et al., J Interv. Cardiol., 6:187-202 (1993); Fischman et al., N Engl J Med, 331:496-501 (1994); Elezi et al., Circulation 98:1875-1880 (1998); Bennett and O'Sullivan, Pharmacol Ther, 91:149-166 (2001); Heckenkamp et al., J Cardiovasc. Surg. (Torino), 43:349-357 (2002)).

Prevention of restenosis after successful PTCA remains one of the most challenging tasks in the treatment of obstructive coronary artery disease. Attempts to ameliorate this proliferative response involve the use coronary stents, which have significantly improved both short term and long term outcome following interventional coronary revascularization procedures. Despite a reduction in restenosis rate with stent deployment, restenosis still occurs in 15-30% of patients within 6 months (Fischman et al., N Engl J Med, 331:496-501 (1994); Elezi et al., Circulation, 98:1875-1880 (1998)). This incidence of in-stent restenosis is expected to increase as coronary stenting is becoming more frequent and is used in less ideal lesions. The disclosed LYST inhibitors can be used to treat or reduce restenosis, abdominal adhesions and scarring following angioplasty.

### Transplant Arteriopathy

In some embodiments, the subject has undergone, is undergoing, or will undergo a transplant. Chronic transplant arteriopathy (CTA) is a major cause of late allograft loss after heart or kidney transplantation (Taylor, et al., J. Heart Lung Transplant., 24:945-955 (2005), Burke, et al., Transplantation, 60:1413-1417 (1995); Cornell and Colvin, Curr. Opin. Nephrol Hypertens., 14:229-234 (2005)). Therefore, in some embodiments, the disclosed LYST inhibitors are used to reduce, or inhibit transplant arteriopathy in a transplant recipient.

### Excessive Scarring

The disclosed LYST inhibitors can be used to treat, retard or reduce scarring, including the formation of keloids and abdominal adhesions, for example, following injury, disease or surgical procedures.

Methods of using one or more LYST inhibitors to decrease the amount of blood vessel growth at the site of an injury or surgery are provided. In some embodiments the methods decrease the formation of high density cellular and connective tissue that give rise to scarring, keloids or adhesions. The amount of one or more LYST inhibitors does not prevent wound healing.

### Hypertrophic Scarring

In some embodiments, the disclosed LYST inhibitors are used to treat, reduce, or inhibit development of hypertrophic scars. Excessive scarring can occur when the tissue response is out of proportion with the amount of scar tissue required for normal repair and healing. When a deep wound involves significant loss of dermis, hypertrophic scar tissue can be deposited at the wound site. The scar tissue contains a high density of cells, increased volume of connective tissue and an increased vascular supply resulting from increased number of blood vessels. Hypertrophic scars can occur as a result of defects in the remodeling phase of tissue repair (Ehrlich and Kelley, Plast Reconstr Surg., 90:993-998 (1992)).

The disclosed LYST inhibitors can be used to diminish the development of blood vessels in the area of a wound to reduce or retard the development of hypertrophic scarring.

### Keloids

In some embodiments, the disclosed LYST inhibitors are used to treat, reduce, or inhibit development of Keloid scars. A keloid scar is a raised or thickened scar that exceeds the boundary of the injury and can continue to develop and enlarge over a prolonged period of time. During development of a keloid growth, collagen, used in wound repair overgrows producing a lump many times larger than that of the original scar.

LYST inhibitors can be used to reduce Keloid development associated with skin injuries, for example, ear piercing, laceration, burns, vaccination or inflammatory processes. LYST inhibitors can be applied locally or topically as needed.

### Adhesions

Adhesions are fibrous bands that form between tissues and organs, often as a result of injury during surgery. They may be thought of as internal scar tissues that connect tissues not normally connected, usually across a virtual space such as the peritoneal cavity. Following abdominal surgery or trauma, the production or activity of fibrinolytic enzymes can be compromised because of injury, and fibrinous adhesion develops. If this is allowed to happen, tissue repair cells such as macrophages, fibroblasts and other blood vessel cells penetrate the fibrinous adhesion to deposit collagen and other matrix substances to form a permanent fibrous adhesion.

Exemplary adhesions that can be treated or reduced by the disclosed LYST inhibitors include abdominal adhesions, pelvic adhesions, including pelvic adhesions that result from endometriosis, pericardial adhesions, peridural adhesions and peritendinous adhesions. Symptoms of adhesions can include abdominal pain, blockages, chronic pelvic pain, cramping, nausea, limited flexibility and inflammation or swelling at the site of the adhesion.

### Other Fibroproliferative Diseases

In some embodiments, the compositions comprising one or more LYST inhibitors can be applied to treat a fibroproliferative disease.

Fibrosis is the deposition of excess fibrous connective tissue in an organ or tissue in response to inflammation and/or damage (Wynn, Nat Rev Immunol., 4(8): 583-594 (2004)). The repair of damaged tissues involves the production of extracellular matrix components at the site of tissue injury. Macrophages and damaged tissues release cytokines and TGF beta which stimulate cells to lay down connective tissue, including collagen and glycosaminoglycans. However, dysregulation of this process can lead to excessive deposition of this connective tissue, which can obliterate the structure and function of underlying organs, leading to the pathology of fibrotic disease. Fibrosis can occur in many tissues within the body, including the liver, lungs, heart and kidney. Methods of treating major-organ fibrosis and fibroproliferative disorders are provided.

### Liver Cirrhosis

Fibrosis of the liver leads to liver damage as hepatocytes are replaced by non-functional scar tissue in a process known as cirrhosis (Masuoka, et al. Ann NY Acad. Sci., 1281:106-122 (2013)). Liver fibrosis and the resulting cirrhosis represent the final common pathway of virtually all chronic liver diseases and can lead to liver failure, liver cancer, and liver-related death. Liver cirrhosis occurs as scar tissue replaces normal parenchyma. Following acute liver injury (e.g., viral hepatitis), parenchymal cells regenerate and replace necrotic or apoptotic cells in a process associated with an inflammatory response. If hepatic injury persists, the liver regeneration process eventually fails and hepatocytes are substituted with abundant extracellular matrix, including fibrillar collagen. Advanced fibrosis is characterized by an accumulation of extracellular matrix proteins rich in fibrillar collagens (predominantly collagen I and collagen III) (reviewed in Iredale, J Clin Invest., 117(3):529-548 (2007); Bataller and Brenner, J Clin Invest, 115(2):209-218 (2005)). Inflammatory cells which activate hepatic stellate cells (HSC) to secrete collagen are an important factor in fibrotic liver disease. Increasing monocyte numbers have been associated with disease progression, specifically with the progression from non-cirrhotic to cirrhotic disease.

Methods of treating liver fibrosis in a subject, including administering to the subject an effective amount of one or more LYST inhibitors to reduce, decrease, limit or prevent one or more symptoms of liver disease relative to an untreated control subject are disclosed. Typical symptoms of liver diseases include, but are not limited to increased abdominal mass, fatigue, abdominal pain, cachexia, jaundice, obstructive syndromes including lymphatic blockage and accumulation of ascites, anemia and back pain (Sun, et al., Clin J. Oncol. Nurs., 12:759-766 (2008)). In some disclosures, one or more LYST inhibitors improve liver function, reduce inflammation, reduce fibrosis, or a combination thereof in a subject with liver disease relative to an untreated control subject.

### Pulmonary Fibrosis

Pulmonary fibrosis is characterized by the deposition of excess fibrous tissue in the lungs that causes marked architectural distortion and loss of alveolar spaces, leading to organ failure and ultimately death from respiratory failure. Pulmonary fibrosis involves gradual exchange of normal lung parenchyma with fibrotic tissue, causing irreversible decrease in oxygen diffusion capacity.

Pulmonary fibrosis (also known as cryptogenic fibrosing alveolitis) is associated with interstitial lung diseases (ILD) such as connective tissue diseases or chronic inflammatory disease (e.g., rheumatoid arthritis), infections, idiopathic lung disease and malignancies. Environmental exposure to inhaled toxins, medications and radiation therapy has also been associated with pulmonary fibrosis.

Methods of treating pulmonary fibrosis in a subject, including administering to the subject an effective amount of one or more LYST inhibitors to reduce, decrease or limit one or more symptoms of pulmonary fibrosis relative to an untreated control subject are provided. Typical symptoms include chronic dry cough, shortness of breath, fatigue and weakness, chest discomfort, loss of appetite and weight loss. In some embodiments, the composition comprising one or more LYST inhibitors improve respiratory function, reduce inflammation, reduce fibrosis, or a combination thereof in a subject with pulmonary fibrosis relative to an untreated control subject.

### Other Fibrotic Diseases

Methods of treating diseases characterized by excessive fibrosis in a subject, including administering to the subject an effective amount of one or more LYST inhibitors to reduce, decrease or limit one or more symptoms of excessive fibrosis relative to an untreated control subject are provided. Exemplary disease and disorders that can be treated by the disclosed methods include nephrosclerosis, scleroderma, Sharp's syndrome, Neurofibromatosis, myelofibrosis, systemic sclerosis, Dupuytren's contracture and macular degeneration. In addition, methods for the treatment of scarring and fibrosis associated with surgical complications, chemotherapeutic, or other drug-induced fibrosis, radiation-induced fibrosis, fibrosis resulting from injury and burns are provided.

### Diseases associated with platelet function

Methods of treating diseases characterized by excessive or deleterious platelet activation and/or platelet derived growth factor (PDGF) mediated signaling are provided. Because platelets play a critical role in the blood clotting process, therapies directed against platelets are some of the most important in the battle against vascular diseases of the heart and brain. Inappropriate or excessive activation of platelets can give rise to clot formation within an intact vessel (thrombosis). A clot that breaks free and begins to travel around the body (thromboembolism) can cause vessel obstruction or occlusion, blood stasis, ischemia, stroke and/or death.

It has been established that reduced LYST leads to reduced platelet activation and reduced expression of PDGF (see Example 7). Accordingly, LYST-inhibitors can be used as anti-platelet drugs, to reduce deleterious platelet aggregation and thrombus formation. Methods of treating diseases characterized by deleterious or excessive platelet activation in a subject, including administering to the subject an effective amount of one or more LYST inhibitors to reduce, decrease or limit one or more symptoms of deleterious or excessive platelet aggregation relative to an untreated control subject are provided. Thus, LYST inhibitors can be used to treat diseases or disorders associated with undesirable or deleterious platelet function. Examples of disorders resulting from inappropriate/deleterious activation of platelets include platelet hyper-aggregation or increased mean platelet volume (MPV) and thrombocytosisl arterial thrombosis; venous thrombosis (Budd-Chiari syndrome; Cavernous sinus thrombosis; Cerebral venous sinus thrombosis; deep vein thrombosis; Paget-Schroetter disease; portal vein thrombosis; jugular vein thrombosis; renal vein thrombosis); and microcirculatory thrombosis. Arterial thrombosis can partially or completely obstruct the flow of blood causing downstream ischemia, stroke or myocardial infarction.

Increased or uncontrolled over-expression of platelet derived growth factor (PDGF) and uncontrolled PDGF signaling has been implicated in the growth, angiogenesis and metastasis of cancer cells as well as diseases associated with such as development, survival and metastasis of tumors (reviewed in Andrae, et al., Genes Dev., 22:pp1276-1312 (2008)). Inhibition of PDGF has been shown to reduce glioma cell growth. Several inhibitors of PDGFR-mediated signaling, such as imatinib, have entered clinical trials for treatment of a variety of malignancies. Accordingly, one or more LYST inhibitors can be used to treat any disease associated with increased or up-regulated expression of PDGF and/or uncontrolled PDGF signaling. Exemplary diseases include cancers such as glioblastomas (e.g., anaplastic oligodendrogliomas) and sarcomas (e.g., dermal sarcoma; esophageal squamous cell sarcomas), as well as retinal vascular disease (e.g., ischemic retinopathies).

### B. Treatments

Inhibition of LYST can be used as a therapeutic mechanism through either local or systemic delivery. In some embodiments, the compositions are administered systemically. Delivery vehicles can be selected and used to target the inhibitors to a particular location or cell type. In other embodiments, the inhibitors are directly administered to the vasculature using a device or graft, such as those discussed above. In further embodiments, the route of administration targets the inhibitors directly to a specific organ or to the local site of injury.

It has been established that the processes of vascular proliferation and inflammation are linked. It is believed that LYST moderates immune processes that contribute to proliferative disorders, such as the restenosis process. Early pharmacological intervention can preclude chronic therapy and any potentially adverse side effects associated with chronic therapy. For example, the compositions disclosed herein can reduce neointima formation, but allow neo-tissue growth to occur. Methods of treatment of diseases and disorders using the disclosed LYST inhibitors optionally including a delivery vehicle are discussed in more detail below.

### Controls

The effect of a LYST inhibitor can be compared to a control. Suitable controls are known in the art and include, for example, untreated cells or an untreated subject. In some embodiments, the control is untreated tissue from the subject that is treated, or from an untreated subject. Preferably the cells or tissue of the control are derived from the same tissue as the treated cells or tissue. In some embodiments, an untreated control subject suffers from the same disease or condition as the treated subject. For example, in some embodiments, one or more of the pharmacological or physiological markers or pathways affected by anti-LYST treatment is compared to the same pharmacological or physiological marker or pathway in untreated control cells or untreated control subjects. For example, anti-LYST treated subjects can be compared to subjects treated with other inhibitors of neointima formation, such as rapamycin. The subjects treated with other inhibitors of neointima formation can have a greater incidence of in post-operative stenosis, or a reduced formation of neo-vascular tissue than do subjects treated with the LYST inhibitors.

Pharmaceutical compositions including one or more LYST inhibitors can be administered in a variety of manners, depending on whether local or systemic treatment is desired, and depending on the area to be treated. For example, the disclosed compositions can be administered intravenously, intraperitoneally, intramuscularly, subcutaneously, intracavity, or transdermally. The compositions may be administered parenterally (e.g., intravenously), by intramuscular injection, by intraperitoneal injection, transdermally, extracorporeally, topically or the like, including topical intranasal administration or administration by inhalation.

Parenteral administration of the composition, if used, is generally characterized by injection. Injectable formulations can be prepared in conventional forms, either as liquid solutions or suspensions, solid forms suitable for solution of suspension in liquid prior to injection, or as emulsions. Administration involving use of a slow release or sustained release system, such that a constant dosage is maintained, is also discussed.

In certain embodiments, the compositions are administered locally, for example, by injection directly into a site to be treated. Local delivery of drugs can reduce side effects or toxicity associated with systemic delivery and can result in enhanced treatment outcome due to an increased localized dose.

Methods of administering the disclosed inhibitors of LYST locally (*i.e.*, *via* local drug delivery, LDD) are provided. In certain embodiments, LYST inhibitors can be administered directly to a treated tissue, such as an artery or vein, without engendering adverse systemic effects. In further embodiments, the compositions are injected or otherwise administered directly to one or more surgical sites. Typically, local injection causes an increased localized concentration of the compositions which is greater than that which can be achieved by systemic administration. In preferred embodiments, the compositions are delivered directly to tissue, prostheses, grafts or medical devices by local injection or topical administration. In some embodiments LYST inhibitors delivered locally result in concentrations that are twice, 10 times, 100 times, 500 times, 1000 times or more than 1000 times greater than that achieved by systemic administration of the same compound. In some embodiments the locally administered LYST inhibitors are steadily released at the site of delivery at a constant rate over a period of time. Preferably, the steady release maintains a desired concentration of the LYST inhibitor at the site of delivery.

The disclosed LYST inhibitors can be administered during a period before, during, or after onset of disease symptoms, or any combination of periods before, during or after onset of one or more disease symptoms. For example, the subject can be administered one or more doses of the composition every 1, 2, 3, 4, 5, 6 7, 14, 21, 28, 35, or 48 days prior to onset of disease symptoms. The subject can be administered one or more doses of the composition every 1, 2, 3, 4, 5, 6, 7, 14, 21, 28, 35, or 48 days after the onset of disease symptoms. In some embodiments, the multiple doses of the compositions are administered before an improvement in disease condition is evident. For example, in some embodiments, the subject receives 1, 2, 3, 4, 5, 6 7, 14, 21, 28, 35, or 48, over a period of 1, 2, 3, 4, 5, 6 7, 14, 21, 28, 35, or 48 days or weeks before an improvement in the disease or condition is evident.

Thus, the disclosed compositions including one or more LYST inhibitors can be administered at different times in relation to a diagnosis, prognosis, surgery or injury depending on the disease or disorder to be treated. The timing of commencement of anti-LYST therapy should be determined based upon the needs of the subject, and can vary from at the time of surgery or injury, to one or more days, weeks or months after surgery or injury. Methods for using formulations for delayed release of LYST inhibitors are provided. In some embodiments, therapy using inhibitors of LYST can be discontinued once vascular neo-tissue growth has occurred.

In some embodiments a single dose of one or more inhibitors of LYST is delivered to a subject as one or more bolus doses to raise the blood concentration of the one or more inhibitors to a desired level. The bolus can be given by any means, such as via injection. The placement of the bolus dose can be varied depending upon the desired effect and the target organ or tissue to be treated. In a particular embodiment, a bolus is given prior to the administration of other dosage forms, such as pulsatile release dosages forms. Thus, the desired blood concentration of one or more LYST inhibitors can be maintained for a desired period of time using a combination of formulations for immediate, delayed or pulsatile release.

In the case of adhesions, the deposition of connective tissue between normal anatomical structures is unnecessary and, thus, preventing the development of vascular tissue is not detrimental. Thus, for reduction of adhesions, LYST inhibitors would typically be applied at the time of surgery or shortly thereafter, for example, within one week. In the case of surgical procedures at risk of neointima formation, for example, stenosis, or restenosis of tissue grafts or stents, one or more LYST inhibitors can be administered at the time of surgery. In the case of injury or surgery involving the skin, the LYST inhibitors can be applied following re-epithelialization of the skin's surface.

### Coating onto Grafts and Devices

One or more LYST inhibitors can be delivered locally by incorporating the one or more inhibitors into a medical device such as a stent or other prosthesis, by loading the inhibitor(s) into or onto a structural or sealing material of the device. The rate of release of the inhibitor(s) may be controlled by a number of methods including varying one or more of the ratio of the absorbable material to the agent, the molecular weight of: the absorbable material, the composition of the inhibitor(s), the composition of the absorbable polymer, the coating thickness, the number of coating layers and their relative thicknesses, the inhibitor concentration, and/or physical or chemical binding or linking of the inhibitor(s) to the device or sealing material. Top coats of polymers and other materials, including absorbable polymers may also be applied to control the rate of release of the one or more inhibitors.

In some embodiments, the amount of LYST inhibitor present on a device or graft tissue can be adjusted by changing the delivery vehicle. The penetration of the inhibitor throughout the tissue can also be adjusted. In this way the amount of drug locally released at the site of implantation can be carefully controlled. Typically, the LYST inhibitor or a delivery vehicle carrying the inhibitor is contacted with a device or graft material *ex vivo.* The contacting can occur in the absence or presence of mild agitation, or other methods known in the art to insure that inhibitor attaches to or penetrates the device or graft tissue. Agitation may be accomplished, for example, by incubation on an orbital shaker, or by vertical rotation, such as by incubation in a vertical carousel of a hybridization oven. The incubation protocol can be varied to affect the positioning of the particles on the device or graft. The amount and localization of attachment of delivery vehicles such as particles to the device or graft can also be varied by varying the type and density of attachment and targeting ligands, such as those described, presented on the vehicle. Exemplary compositions and methods for delivering drugs to vascular grafts *ex vivo* are discussed in U.S. Published Application Nos. 2006/0002971, 2010/0151436, and U.S. Patent No. 7,534,448.

The compositions and methods can be used to locally deliver LYST inhibitors to grafts with, or without the requirement for further invasive procedures, such as placement of a vascular graft or stent.

### C. Dosages and Effective Amounts

In accordance with the present invention, the compositions of LYST inhibitors are administered to a subject in a therapeutically effective amount that is between 0.1 and 1000 mg/kg body weight of a human and does not prevent wound healing. As used herein the term "effective amount" or "therapeutically effective amount" means a dosage sufficient to treat, inhibit, or alleviate one or more symptoms of the disorder being treated or to otherwise provide a desired pharmacologic and/or physiologic effect. The precise dosage will vary according to a variety of factors such as subject-dependent variables (*e.g.*, age, immune system health, *etc.*), the disease or disorder, and the treatment being effected.

For all of the disclosed compounds, as further studies are conducted, information will emerge regarding appropriate dosage levels for treatment of various conditions in various patients, and the ordinary skilled worker, considering the therapeutic context, age, and general health of the recipient, will be able to ascertain proper dosing. The selected dosage depends upon the desired therapeutic effect, on the route of administration, and on the duration of the treatment desired. Generally dosage levels of between 0.001 and 100 mg/kg of body weight daily are administered to mammals, most preferably, humans. Generally, for intravenous injection or infusion, dosage may be lower. Preferably, the compositions are formulated to achieve a LYST inhibitor serum level of between about 1 and about 1000 µM at the site where treatment is desired.

In some embodiments, the LYST inhibitors are effective to prevent the normal biological activities of immune cells, such as macrophages, platelets and NK cells. For example, one or more inhibitors can be in an amount effective to reduce the migratory or chemotactic activity of macrophages.

In one embodiment the one or more LYST inhibitors are in an amount effective to reduce neointima formation in a subject. The amount of one or more LYST inhibitors does not prevent wound healing. Preferably, the amount of one or more LYST inhibitors does not prevent the formation of neotissue at the site of graft implantation in a subject compared to an untreated control. Preferably, the amount of one or more LYST inhibitors is effective to reduce neointima formation and enhance wound healing in a subject compared to an untreated control.

In another embodiment, the one or more LYST inhibitors are in an amount effective to decrease the amount of blood vessel growth at the site of an injury or surgery, to decrease the formation of high density cellular and connective tissue that give rise to fibrosis, scarring, keloids or adhesions. The amount of one or more LYST inhibitors does not prevent wound healing.

In another embodiment, one or more LYST inhibitors are in an amount effective to decrease or inhibit platelet activation. For example, LYST inhibitors can be in an amount effective to inhibit inappropriate platelet aggregation and secretion of chemokines at the site of an injury or surgery. In a further embodiment, the one or more LYST inhibitors are in an amount effective to decrease the amount of Platelet Derived Growth Factor (PDGF) and/or Transforming Growth Factor Beta (TGFβ) produced or secreted by cells in response to activation by thrombin at a the site of an injury or surgery. One or more LYST inhibitors can be effective to reduce the production or secretion of PDGF-A, PDGF-B, PDGF-C, or PDGF-D. Thus, one or more LYST inhibitors can be effective to reduce the amount of biologically active PDGF in the serum. For example, the amount of PDGF-AA, PDGF-BB, PDGF-CC, PDGF-DD or PGDF-AB can be reduced relative to the amount in an untreated control. Accordingly, one or more LYST inhibitors can be effective to reduce or prevent one or more biological activities that occur as a result of PDGF, or as a result of downstream signaling events controlled by PDGF. For example, one or more LYST inhibitors can be effective to reduce or prevent the activation of one or more tyrosine kinase receptors, such as PDGF Receptor α (PDGFRα); PDGF Receptor β (PDGFRβ); and/or PDGF Receptor αβ (PDGFαβ). By reducing or preventing the activity of one or more PDGFs, the LYST inhibitors can reduce or prevent PDGF-mediated induction of several signaling pathways controlling cellular activities including cellular proliferation, chemotaxis and actin reorganization.

In another embodiment, the one or more LYST inhibitors are in an amount effective to decrease the formation of high density cellular and connective tissue that give rise to fibrosis, scarring, keloids or adhesions. The amount of one or more LYST inhibitors does not prevent wound healing.

### D. Combination Therapies

The disclosed compositions, devices, and grafts including LYST inhibitors can be administered alone, or in combination with one or more additional active agent(s), as part of a therapeutic or prophylactic treatment regime. For example, the composition can be administered on the first, second, third, or fourth day, or combinations thereof. The composition can be administered on the same day, or a different day than the second active agent.

The term "combination" or "combined" is used to refer to either concomitant, simultaneous, or sequential administration of two or more agents. Therefore, the combinations can be administered either concomitantly (*e.g.*, as an admixture), separately but simultaneously (*e.g.*, *via* separate intravenous lines into the same subject), or sequentially (*e.g.*, one of the compounds or agents is given first followed by the second).

The additional therapeutic agents can be other anti-neointima agents, chemotherapeutic agents, antibodies, antibiotics, antivirals, steroidal and non-steroidal anti-inflammatories, conventional immunotherapeutic agents, immune-suppressants, cytokines, chemokines and/or growth factors, anti-proliferatives or anti-migration agents designed for treating or preventing neointima formation or restenosis, agents which affect migration and extracellular matrix production, agents which affect platelet deposition or formation of thrombus, and agents that promote vascular healing and re-endothelialization, described in Tanguay et al. Current Status of Biodegradable Stents, Cardiology Clinics, 12:699-713 (1994), J. E. Sousa, P. W. Serruys and M. A. Costa, Circulation 107 (2003) 2274 (Part I), 2283 (Part II), K. J. Salu, J. M. Bosmans, H. Bult and C. J. Vrints, Acta Cardiol 59 (2004) 51.

Exemplary anti-thrombin agents include, but are not limited to, Heparin (including low molecular heparin), R-Hirudin, Hirulog, Argatroban, Efegatran, Tick anticoagulant peptide, and Ppack.

Exemplary antiproliferative agents include, but are not limited to, Paclitaxel (Taxol), QP-2 Vincristin, Methotrexat, Angiopeptin, Mitomycin, BCP 678, Antisense c-myc, ABT 578, Actinomycin-D, RestenASE, 1 - Chlor-deoxyadenosin, PCNA Ribozym, and Celecoxib.

Exemplary agents modulating cell replication/proliferation include targets of rapamycin (TOR) inhibitors (including sirolimus, everolimus and ABT-578), paclitaxel and antineoplastic agents, including alkylating agents (e.g., cyclophosphamide, mechlorethamine, chlorambucil, melphalan, carmustine, lomustine, ifosfamide, procarbazine, dacarbazine, temozolomide, altretamine, cisplatin, carboplatin and oxaliplatin), antitumor antibiotics (*e.g*., bleomycin, actinomycin D, mithramycin, mitomycin C, etoposide, teniposide, amsacrine, topotecan, irinotecan, doxorubicin, daunorubicin, idarubicin, epirubicin, mitoxantrone and mitoxantrone), antimetabolites (*e.g.*, deoxycoformycin, 6-mercaptopurine, 6-thioguanine, azathioprine, 2-chlorodeoxyadenosine, hydroxyurea, methotrexate, 5-fluorouracil, capecitabine, cytosine arabinoside, azacytidine, gemcitabine, fludarabine phosphate and aspariginase), antimitotic agents (*e.g*., vincristine, vinblastine, vinorelbine, docetaxel, estramustine) and molecularly targeted agents (*e.g.*, imatinib, tretinoin, bexarotene, bevacizumab, gemtuzumab ogomicin and denileukin diftitox).

Exemplary anti-restenosis agents include, but are not limited to, immunomodulators such as Sirolimus (Rapamycin), Tacrolimus, Biorest, Mizoribin, Cyclosporin, Interferon .gamma. 1b, Leflunomid, Tranilast, Corticosteroide, Mycophenolic acid and Biphosphonate.

Exemplary anti-migratory agents and extracellular matrix modulators include, but are not limited to Halofuginone, Propyl-hydroxylase-Inhibitors, C-Proteinase-Inhibitors, MMP-Inhibitors, Batimastat, Probucol. Examples of antiplatelet agents include, but are not limited to, heparin.

In some embodiments the additional therapeutic agent is N-3,4-trihydroxybenzamide or a pharmaceutically acceptable salt or ester thereof, didox, imidate, or hydroxyurea as described in U.S. Patent No. 8,029,815.

The additional therapeutic agents can be administered locally or systemically to the subject, or coated or incorporated onto, or into a device or graft. The additional therapeutic reagents can be administered by the same, or by different routes and by different means. For example, one or more LYST inhibitors can be delivered locally combined with one or more of paclitaxel, taxotere and other taxoid compounds, methotrexate, anthracyclines such as doxorubicin, everolimus, serolimus, rapamycin or rapamycin derivatives delivered by different means, such as systemically.

In further embodiments, one or more LYST inhibitors may be used to reduce the unwanted side-effects associated with the use of one or more additional therapeutic agents. For example, one or more LYST inhibitors can be used with the anti-neoplastic agent bleomycin, to reduce pulmonary fibrosis associated with bleomycin.

The present invention will be further understood by reference to the following non-limiting examples.

### Examples

### Example 1: The beige mutation reduces stenosis in tissue grafts

### Methods and Materials

Tissue engineered vascular grafts (TEVG) were developed by seeding autologous bone marrow-derived mononuclear cells onto a biodegradable tubular scaffold fabricated from a polyglycolic acid-fiber tube coated with a 50:50 copolymer of poly lactic acid and polycaprolactone.

A murine model for evaluating TEVG function was developed in an effort to elucidate the cellular and molecular mechanisms underlying the formation of TEVG. The inferior vena cava (IVC) interposition graft model is a validated model for investigating the use of vascular grafts in a low pressure, high flow circulatory system similar to the Fontan circulation. This model was used extensively to study neotissue formation in the TEVG.

A biodegradable conduit graft composed of PGA-PCL/LA was implanted into the inferior vena cava of wild-type mice and beige mutant mice, respectively, without cell seeding. Grafts were explanted 2 weeks after surgery, sectioned and measured for the diameter of the vessel and assessed for patency.

### Results

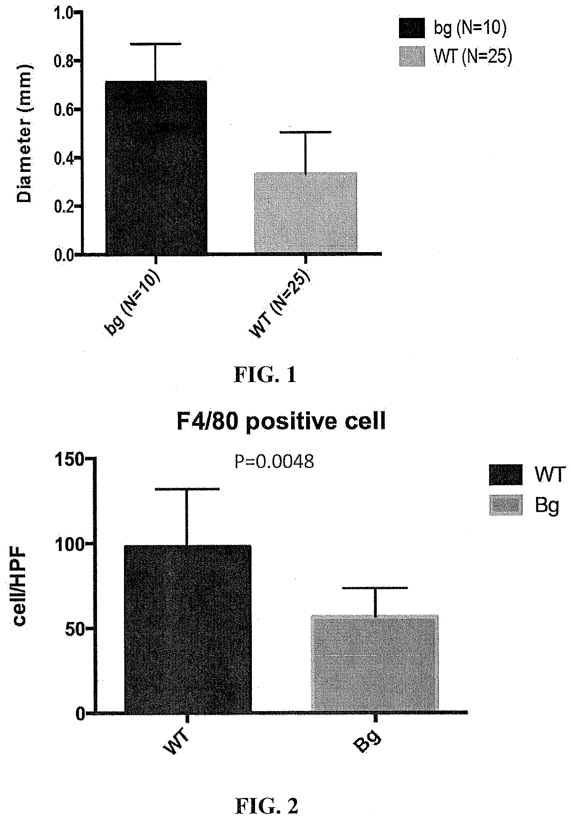

Significant variation in the stenosis rates was observed when TEVG were implanted as IVC interposition grafts, depending on the strain of the mouse. Specifically, it was noted that TEVG implanted in mice with a beige mutation had exceptionally low rates of stenosis. The patency and luminal diameter of TEVG implanted as IVC interposition grafts were compared in both C57B6 (wild-type mice) (N=25) and beige mutants (N=10) (Table 1). The wild-type mice exhibited a 80% stenosis with luminal diameters measuring 0.33 mm whereas the beige mice exhibited a 10% stenosis rate with luminal diameters measuring 0.71 mm (T-test <0.001 and Chi-square 0.0002) (Figure 1).

**Table 1: Differences in the patency and luminal diameters of TEVG explanted from Beige (Bg) and wild-type (Wt) mice.**

| **Gunze 21G** | **n** | **Diameter** | **SD** | **Patent (>0.45mm)** | **Patency (%)** |
|---|---|---|---|---|---|
| Beige | 10 | 0.71 | 0.16 | 9 | 90 |
| Lyst-tm1b | 5 | 0.73 | 0.17 | 5 | 100 |
| WT | 25 | 0.33 | 0.17 | 5 | 20 |

### Example 2: The beige mutation reduces macrophage infiltration into tissue grafts

### Methods and Materials

It has previously been demonstrated using the murine model that vascular neotissue formation arises from cells derived from the host and that the process of vascular neotissue formation is orchestrated by the immune system, specifically host-derived macrophages. Excessive macrophage infiltration leads to stenosis, while inhibition of macrophage infiltration prevents neotissue formation.

The number of macrophages in the explanted grafts described above in Example 1 was measured for both beige and wild type mice. Immunohistochemistry was carried out on tissue sections of each graft to characterize the number and morphology of endothelial cells in each group, using an antibody specific for the CD31 cell marker.

### Results

The TEVG implanted in the beige mice exhibited significantly less macrophage infiltration than TEVG implanted in the wild type mice at 2 weeks after surgery. See Figure 2. Notably, immunohistochemistry for endothelial cells (using an antibody specific for CD 31) did not show any difference between graft sections from wild-type and beige mice, indicating that neotissue formation was similar in both groups.

### Example 3: The beige phenotype arises from a mutation in the LYST gene

### Methods and Materials

The beige mutation arises from a spontaneous mutation of the LYST gene (Figure 3). For analysis of protein expression, 6 different primers, designated LYST1, LYST3, LYST4, LYST5 and LYST6, respectively, were designed to cover the complete LYST gene region. The LYST-5 primer covered the area of exon 52 that included the amino acid mutation identified in the beige mouse genotype. Raw cells were used as a standard to calculate relative mRNA levels.

### Results

The specific mutation responsible for the beige phenotype in Beige mice was identified. The full length LYST gene encodes a 3801 amino acid polypeptide. The mutation of LYST occurred on exon 52 resulting in deletion of the Amino acid isoleucine (ile3741del) (Figure 3).

Molecular analyses demonstrated that there was no significant difference in expression of mRNA between both groups. Despite equivalent levels of transcription of the wild-type and mutant proteins, there was no translation of the mutant protein.

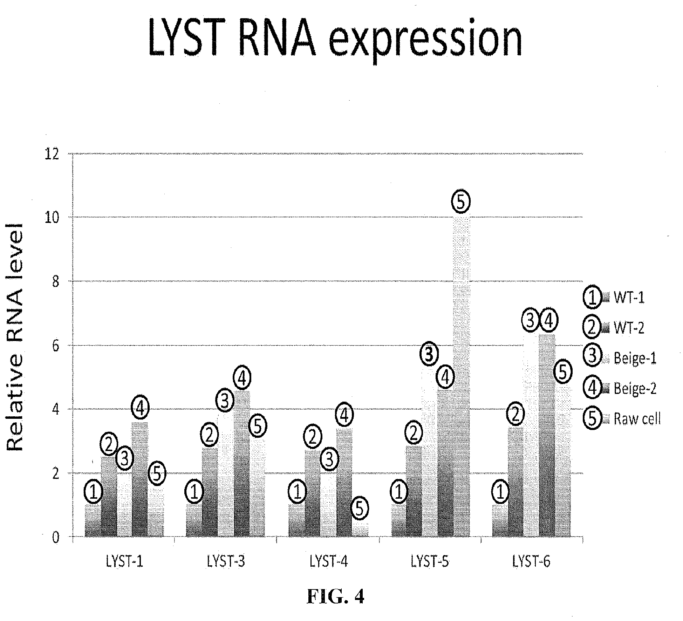

Further, LYST protein expression was depleted in beige mutant mouse by immunohistochemistry and western blotting (Figure 4). These results indicated that LYST gene mutation in beige mouse affect protein modification level after translation from gene.

### Example 4: The beige mutation in the LYST gene alters immune processes

### Methods and Materials

The hypothesis that the beige mutation inhibits the formation of TEVG stenosis by modulating the immune system was tested. Bone marrow was transplanted from C57B6 (wild-type) mice into beige mutant mice, and vice versa. FACS analyses were carried out to determine the percentage of LYST monocytes in Beige and wild-type mice. In addition, the ability of platelet activation upon stimulation with fibrin was reduced in beige mutant mice compared with wild-type.

### Results

Significant inhibition of TEVG stenosis was demonstrated in wild-type mice transplanted with bone marrow from the beige mice. Similarly, when TEVG were implanted in beige mice transplanted with the bone marrow from wild-type mice they exhibited high rates of TEVG stenosis. These findings indicated the beige mutation inhibits TEVG stenosis via its effect on the immune system. FACS analysis of monocytes and macrophages from Wt and Beige mice, respectively, demonstrated difference in the phenotype population. A series of scatter dot plots showing representative results of analytical flow cytometry show the proportion of monocyte cells stained for CD115 (Y-axis) and CD11b (X-axis) from WT3, WT4, bg3 and bg4 mice, respectively. The distribution of cells that are stained for LYC6 and F4/80 from WT3, WT4, bg3 and bg4 mice, respectively, were also determined.. The percentage of cells designated as LYC6 "Hi" and LYC6 "lo" was calculated. Results are representative of peritoneal macrophages 3 days after stimulation with thioglycolate, gated to exclude dead cells and debris.

Scatter dot plots showed representative results of analytical flow cytometry, plotting cells stained for PE-A (Y-axis) against FITC-A fluorescence (X-axis) from WT mice and Beige mice, respectively. Cells were gated to exclude dead cells and debris following platelet activation following stimulation with fibrin. The number of LY6C "hi" monocytes was higher in wild type group (WT3, WT4), whereas the number of Ly6C "low" monocytes was higher in beige group (bg 3, bg) in peritoneal macrophage at 3 days after stimulation with thioglycolate.

FACS analysis also demonstrated that the ability of platelet activation upon stimulation of cells with fibrin was reduced in beige mutant mice, as compared with wild-type mice.

### Example 5: LYST-mediated immunomodulation can be achieved using an antibody to the LYST protein

### Methods and Materials

The effects of blocking the LYST protein were investigated using an anti-LYST antibody. C57B6 mice were treated with the anti-LYST antibody and then implanted with TEVG. Anti- LYST antibody was injected into peritoneum of wild type mouse in each of three different treatment groups (0 mg/kg (control), 10 mg/kg, or 50 mg/kg) at 1 day before surgery, as well as 1 week after surgery. Mice were sacrificed and graft and spleen was explanted at 2 weeks after surgery. Histology was carried out using Hematoxylin and eosin staining (HE stain) of the graft. Spleen tissue was analyzed by Western blotting using an antibody specific for the LYST protein to determine the relative effective amount of LYST protein in each treatment group.

### Results

The systemic injection of anti- LYST antibody showed improvement of graft patency in dose dependent fashion. A noted inhibition of macrophage infiltration and decreased TEVG stenosis was observed. This dose-dependent increase in patency was accompanied by a dose-dependent reduction in the presence of the LYST protein within spleen tissue, as determined by immunohistochemistry. Thus, a method of immunomodulation that results in improved neotissue formation and inhibition of the formation of TEVG stenosis has been established.

### Example 6: Transgenic mice lacking a functional Lyst gene do not exhibit stenosis of TEVG

### Methods and Materials

C57BL/6 transgenic mice lacking a functional LYST protein were generated. These mice were designated C57BL/6 Lyst^{tm1b}. The technology used to generate this knockout strain uses a LacZ reporter. PCR for LacZ confirmed the knockout model.

To determine whether C57BL/6Lyst^{tm1b} knockout mice would develop stenosis following TEVG implantation, 5 C57BL/6 Lyst^{tm1b} mice and 5 C57BL/6 wild type mice were implanted with unseeded TEVG. The TEVG were explanted and examined 2 weeks following implantation.

### Results

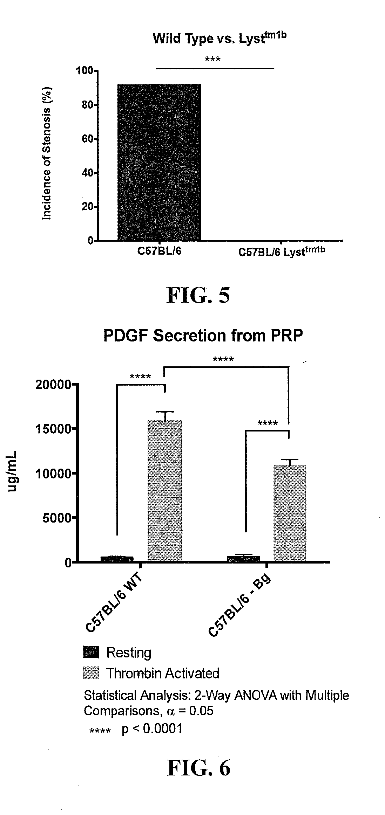

100% of unseeded tissue engineered vascular grafts (TEVG) implanted in Lyst^{tm1b} knockout mice (n=5) were patent after 2-week implantation (see Figure 5; Table 1). In contrast, C57BL/6 wild type mice exhibited approximately 90% incidence of stenosis.

### Example 7: Lyst modulates platelet function in response to activation Methods and Materials

The Lyst protein is understood to be involved in platelet function, and Chediak Higashi Syndrome, the human correlate, is characterized as a "platelet storage pool deficiency." Platelet rich plasma (PRP) was obtained from Beige (Bg) and Wild Type (WT) mice. Samples were activated by thrombin and total concentrations of secreted PDGF (platelet derived growth factor) from each group (n=7) were compared to resting, non-activated controls (n=5).

### Results

In both the activated and resting groups, thrombin activation resulted in significantly greater concentrations of PDGF. However, the PDGF secretion from activated platelets was significantly decreased in the Beige group when compared to Wild Type (see Figure 6). Thus, reduction of Lyst activity also reduced PDGF, indicating that agents which reduce Lyst activity can also function as anti-platelet agents.

## Claims

1. A pharmaceutical composition for use in a method of reducing macrophage infiltration, scar formation, or stenosis at a site of graft implantation in a subject comprising:
a) one or more inhibitors of lysosomal trafficking regulator (LYST) in an amount effective to reduce macrophage infiltration, natural killer cell activation, and/or platelet activation in the subject, and
b) a physiologically acceptable carrier,
wherein the composition is administered to the subject prior to surgery, at the time of surgery, and/or within days after surgery, and
wherein the amount of one or more inhibitors of LYST is between 0.1 and 1000 mg/kg body weight of a human and does not prevent wound healing.

2. The pharmaceutical composition for use in a method of claim 1, wherein the composition is administered prior to surgery.

3. The pharmaceutical composition for use in a method of claim 1 or 2, wherein the amount of one or more inhibitors of LYST does not prevent vascular neotissue formation at the site of graft implantation in the subject.

4. The pharmaceutical composition for use in a method of any one of claims 1-3, wherein the composition is used to reduce the expression of platelet derived growth factor, transforming growth factor beta, or any combination thereof in a subject relative to an untreated control subject.

5. The pharmaceutical composition for use in a method of claim 1 in a dosage effective to reduce macrophage infiltration.

6. The pharmaceutical composition for use in a method of claim 1 in a dosage effective to reduce platelet activation.

7. The pharmaceutical composition for use in a method of claim 1 wherein one or more LYST inhibitors are antibodies, antibody fragments, or proteins having the binding specificity of an anti-LYST antibody.

8. The pharmaceutical composition for use in a method of claim 1 wherein one or more LYST inhibitors is a functional nucleic acid selected from the group consisting of an antisense molecule, siRNA, miRNA, aptamers, ribozymes, triplex forming molecules, RNAi, and external guide sequences.

9. The pharmaceutical composition for use in a method of claim 8 wherein one or more functional nucleic acids are expressed from an expression vector.

10. The pharmaceutical composition for use in a method of claim 1 further comprising a delivery vehicle selected from the group consisting of nanoparticles, microparticles, micelles, emulsions, synthetic lipoprotein particles, liposomes, carbon nanotubes, gels, and coatings.

11. The pharmaceutical composition for use in a method of claim 1 further comprising one or more additional therapeutic agents selected from the group consisting of other anti-neointima agents, chemotherapeutic agents, steroidal and non-steroidal anti-inflammatories, conventional immunotherapeutic agents, immune-suppressants, cytokines, chemokines, and growth factors.

12. A pharmaceutical composition for use in a method of reducing platelet activation and arterial or venous thrombosis in a subject with a graft, the method comprising administering to a subject in need thereof the composition of any one of claims 1-11 before and following graft implantation.

13. The pharmaceutical composition for use in a method of claim 12, wherein the composition is used to reduce the expression of platelet derived growth factor, transforming growth factor beta, or any combination thereof in a subject relative to an untreated control subject.

## Patentansprüche

1. Pharmazeutische Zusammensetzung zur Verwendung in einem Verfahren zum Verringern einer Infiltration von Makrophagen, einer Narbenbildung oder einer Stenose an einer Stelle einer Transplantatimplantation bei einem Subjekt, die Folgendes umfasst:
a) einen oder mehrere Inhibitoren des Lysosomal Trafficking Regulators (LYST) in einer Menge, die wirksam ist, um die Infiltration von Makrophagen, eine Aktivierung natürlicher Killerzellen und/oder eine Aktivierung von Blutplättchen bei dem Subjekt zu verringern, und
b) eine physiologisch unbedenkliche Trägersubstanz,
wobei die Zusammensetzung dem Subjekt vor einer Operation, zum Zeitpunkt der Operation und/oder innerhalb von Tagen nach der Operation verabreicht wird, und
wobei die Menge eines oder mehrerer Inhibitoren von LYST zwischen 0,1 und 1000 mg/kg Körpergewicht eines Menschen liegt und eine Wundheilung nicht verhindert.

2. Pharmazeutische Zusammensetzung zur Verwendung in einem Verfahren nach Anspruch 1, wobei die Zusammensetzung vor der Operation verabreicht wird.

3. Pharmazeutische Zusammensetzung zur Verwendung in einem Verfahren nach Anspruch 1 oder 2, wobei die Menge eines oder mehrerer Inhibitoren von LYST eine Ausbildung von vaskulärem Neugewebe an der Stelle der Transplantatimplantation bei dem Subjekt nicht verhindert.

4. Pharmazeutische Zusammensetzung zur Verwendung in einem Verfahren nach einem der Ansprüche 1-3, wobei die Zusammensetzung verwendet wird, um die Expression eines von Blutplättchen abstammenden Wachstumsfaktors, eines transformierenden Wachstumsfaktors Beta oder einer beliebigen Kombination davon bei einem Subjekt relativ zu einem unbehandelten Kontrollsubjekt zu verringern.

5. Pharmazeutische Zusammensetzung zur Verwendung in einem Verfahren nach Anspruch 1 in einer Dosierung, die wirksam ist, um die Infiltration von Makrophagen zu verringern.

6. Pharmazeutische Zusammensetzung zur Verwendung in einem Verfahren nach Anspruch 1 in einer Dosierung, die wirksam ist, um die Aktivierung von Blutplättchen zu verringern.

7. Pharmazeutische Zusammensetzung zur Verwendung in einem Verfahren nach Anspruch 1, wobei ein oder mehrere LYST-Inhibitoren Antikörper, Antikörperfragmente oder Proteine sind, die die Bindungsspezifität eines Anti-LYST-Antikörpers aufweisen.

8. Pharmazeutische Zusammensetzung zur Verwendung in einem Verfahren nach Anspruch 1, wobei ein oder mehrere LYST-Inhibitoren eine funktionelle Nukleinsäure sind, die aus der Gruppe ausgewählt ist, die aus Folgendem besteht: einem Antisense-Molekül, siRNA, miRNA, Aptameren, Ribozymen, triplexausbildenden Molekülen, RNAi und externen Leitsequenzen.

9. Pharmazeutische Zusammensetzung zur Verwendung in einem Verfahren nach Anspruch 8, wobei eine oder mehrere funktionelle Nukleinsäuren aus einem Expressionsvektor exprimiert werden.

10. Pharmazeutische Zusammensetzung zur Verwendung in einem Verfahren nach Anspruch 1, die ferner einen Abgabeträgerstoff umfasst, der aus der Gruppe ausgewählt ist, die aus Folgendem besteht: Nanopartikeln, Mikropartikeln, Mizellen, Emulsionen, synthetischen Lipoproteinpartikeln, Liposomen, Kohlenstoffnanoröhren, Gelen und Beschichtungen.

11. Pharmazeutische Zusammensetzung zur Verwendung in einem Verfahren nach Anspruch 1, die ferner ein oder mehrere zusätzliche therapeutische Mittel umfasst, die aus der Gruppe ausgewählt sind, die aus Folgendem besteht: anderen Anti-Neointima-Mitteln, chemotherapeutischen Mitteln, steroidalen und nichtsteroidalen Entzündungshemmern, herkömmlichen immunotherapeutischen Mitteln, Immunsuppressiva, Zytokinen, Chemokinen und Wachstumsfaktoren.

12. Pharmazeutische Zusammensetzung zur Verwendung in einem Verfahren zum Verringern der Aktivierung von Blutplättchen und einer arteriellen oder venösen Thrombose bei einem Subjekt mit einem Transplantat, wobei das Verfahren ein Verabreichen der Zusammensetzung nach einem der Ansprüche 1-11 vor und nach der Transplantatimplantation an ein Subjekt umfasst, das dies benötigt.

13. Pharmazeutische Zusammensetzung zur Verwendung in einem Verfahren nach Anspruch 12, wobei die Zusammensetzung verwendet wird, um die Expression des von Blutplättchen abstammenden Wachstumsfaktors, des transformierenden Wachstumsfaktors Beta oder einer beliebigen Kombination davon bei einem Subjekt relativ zu einem unbehandelten Kontrollsubjekt zu reduzieren.

## Revendications

1. Composition pharmaceutique destinée à être utilisée dans un procédé de réduction de l'infiltration de macrophages, de la formation de cicatrices ou de la sténose au niveau d'un site d'implantation de greffe chez un sujet comprenant :
a) un ou plusieurs inhibiteurs du régulateur du trafic lysosomal (LYST) en une quantité efficace pour réduire l'infiltration de macrophages, l'activation des cellules tueuses naturelles et/ou l'activation de plaquettes chez le sujet, et
b) un support pharmaceutiquement acceptable,
la composition étant administrée au sujet avant la chirurgie, au moment de la chirurgie et/ou dans les jours après la chirurgie, et
la quantité d'un ou plusieurs inhibiteurs de LYST étant comprise entre 0,1 et 1000 mg/kg de poids corporel d'un être humain et n'empêchant pas la cicatrisation de la plaie.

2. Composition pharmaceutique destinée à être utilisée dans un procédé selon la revendication 1, la composition étant administrée avant la chirurgie.

3. Composition pharmaceutique destinée à être utilisée dans un procédé selon la revendication 1 ou 2, la quantité d'un ou plusieurs inhibiteurs de LYST n'empêchant pas la formation de néotissu vasculaire au site d'implantation du greffon chez le sujet.

4. Composition pharmaceutique à utiliser dans un procédé selon l'une quelconque des revendications 1 à 3, la composition étant utilisée pour réduire l'expression du facteur de croissance dérivé des plaquettes, du facteur bêta de transformation de croissance ou de toute combinaison de ceux-ci chez un sujet par rapport à un sujet témoin non-traité.

5. Composition pharmaceutique destinée à être utilisée dans un procédé selon la revendication 1 en une dose efficace pour réduire l'infiltration de macrophages.

6. Composition pharmaceutique destinée à être utilisée dans un procédé selon la revendication 1 en une dose efficace pour réduire l'activation de plaquettes.

7. Composition pharmaceutique destinée à être utilisée dans un procédé selon la revendication 1, un ou plusieurs inhibiteurs de LYST étant des anticorps, des fragments d'anticorps ou des protéines ayant la spécificité de liaison d'un anticorps anti-LYST.

8. Composition pharmaceutique destinée à être utilisée dans un procédé selon la revendication 1, un ou plusieurs inhibiteurs de LYST étant un acide nucléique fonctionnel choisi dans le groupe constitué par une molécule antisens, un ARNsi, un ARNmi, des aptamères, des ribozymes, des molécules formant un triplex, une ARNi, des séquences de guide externe.

9. Composition pharmaceutique destinée à être utilisée dans un procédé selon la revendication 8, un ou plusieurs acides nucléiques fonctionnels étant exprimés à partir d'un vecteur d'expression.

10. Composition pharmaceutique destinée à être utilisée dans un procédé selon la revendication 1 comprenant en outre un véhicule d'administration choisi dans le groupe constitué de nanoparticules, microparticules, micelles, émulsions, particules de lipoprotéines synthétiques, liposomes, nanotubes de carbone, gels et revêtements.

11. Composition pharmaceutique destinée à être utilisée dans un procédé selon la revendication 1, comprenant en outre un ou plusieurs agents thérapeutiques supplémentaires choisis dans le groupe comprenant d'autres agents anti-néointima, des agents chimiothérapeutiques, des anti-inflammatoires stéroïdiens et non-stéroïdiens, des agents immunothérapeutiques conventionnels, des immunosuppresseurs, cytokines, chimiokines et facteurs de croissance.

12. Composition pharmaceutique destinée à être utilisée dans un procédé de réduction de l'activation de plaquettes et de la thrombose artérielle ou veineuse chez un sujet avec une greffe, le procédé comprenant l'administration de la composition à un sujet en ayant besoin selon l'une quelconque des revendications 1 à 11 avant
et après l'implantation du greffon.

13. Composition pharmaceutique destinée à être utilisée dans un procédé selon la revendication 12, la
composition étant utilisée pour réduire l'expression du facteur de croissance dérivé de plaquettes, du facteur bêta de transformation de croissance ou de toute combinaison de ceux-ci chez un sujet par rapport à un sujet témoin non-traité.
